(19) Europäisches Patentamt
European Patent Office
Office européen des brevets

(11) EP 3 110 828 B1

(12) **EUROPEAN PATENT SPECIFICATION**

(45) Date of publication and mention of the grant of the patent:
**09.12.2020 Bulletin 2020/50**

(21) Application number: **15715810.6**

(22) Date of filing: **27.02.2015**

(51) Int Cl.:
***C07K 1/04*** *(2006.01)*

(86) International application number:
**PCT/HU2015/000022**

(87) International publication number:
**WO 2015/128687 (03.09.2015 Gazette 2015/35)**

(54) **CONTINUOUS FLOW PEPTIDE SYNTHESIS**

DURCHLAUF-PEPTIDSYNTHESE

SYNTHÈSE PEPTIDIQUE À FLUX CONTINU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.02.2014 HU 1400114**

(43) Date of publication of application:
**04.01.2017 Bulletin 2017/01**

(73) Proprietor: **Szegedi Tudományegyetem**
**6720 Szeged (HU)**

(72) Inventors:
- **FÜLÖP, Ferenc**
**6725 Szeged (HU)**
- **MÁNDITY, István**
**6726 Szeged (HU)**
- **OLASZ, Balázs**
**6723 Szeged (HU)**

(74) Representative: **Szentpéteri, Zsolt**
**SBGK Patent and Law Offices**
**Andrassy ut 113**
**1062 Budapest (HU)**

(56) References cited:
**EP-A2- 1 491 552** **WO-A1-82/03077**
**US-A- 4 192 798** **US-A- 4 861 866**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 110 828 B1

Printed by Jouve, 75001 PARIS (FR)

## Description

## BACKGROUND OF THE INVENTION

### 1. Technical Field of the Invention

The invention relates to a continuous flow chemical method for the solid phase synthesis of peptides and enantiomers, diastereomers, stereoisomers, mixtures, salts and/or derivatives thereof.

### 2. Descriprion of the Prior Art

Biopolymers constituting the basis of life, like oligo- and polysaccharides, oligo- and polynucleotides, and oligo- and polypeptides are of considerable interest. Peptides and proteins are polymers built from amino acids. Due to their important biological effects, this field was intensively investigated at the beginning of the 20th century. First the effects of isolates have been examined, later syntheses were performed in liquid phase to obtain peptides (Bernd Groner: Peptides as Drugs: Discovery and Development Wiley-VCH 2009).

Peptide drugs, like vasopressin, goserelin, exenatide, octreotide, etc. form one of the practical applications of peptides (C.M.B. Edwards, M.A. Cohen, S.R. Bloom Q. J. Med. 1999; 92, 1-4; Cynthia L. Stevenson Current Pharmaceutical Biotechnology, 2009, 10, 122- -137). Proteins, in the form of vaccines containing monoclonal antibodies, are applied in the therapy of cancer. An example for this application is represented by Trastuzumab (Shak, S SEMINARS IN ONCOLOGY 1999, 26, 71-77). Furthermore, the synthesis and application of modified and radiolabelled peptides became important too (Fischer, G; Schirrmacher, R; Wangler, B; Wangler, C CHEMMEDCHEM 2013, 8, 883-890). Some peptides can be connected to diseases, like β-amyloid peptide in the case of Alzheimer disease. The synthesis of said peptides accelerated the investigation of the disease itself (Bacsa, Bernadett; Bosze, Szilvia; Kappe, C. Oliver JOURNAL OF ORGANIC CHEMISTRY 2010, 75,2103-2106).

Due to the difficulties of the synthetic methods, initially peptides isolated from organs of animals have been used in practice; later, nowadays, peptides are synthesized by biotechnological methods utilizing genetic engineering, since longer sequences can not yet be synthesized in economically efficient way using the existing peptide synthesis technologies. Still nowadays a constant need exsists for a fast, efficient peptide synthesis method (V.R. Pattabiraman, J. W. Bode: Nature 480, 471-479, 2011). In modern peptide research unnatural amino acid derivatives are growingly used giving often rise to important increase in expenses of the peptide synthesis, thus the reduction of the amino acid excess used in the peptide synthesis would lead to remarkable cut in costs and to reduced loading of the environment as well.

As presented above the realization of the peptide synthesis has primordial importance in the research and also in the practice [Murray Goodman, Claudio Toniolo, Luis Moroder: Synthesis of Peptides and Peptidomimetics (Methods in Organic Chemistry) Houben-Weyl, 2002; Andrew B. Hughes: Amino Acids, Peptides and Proteins in Organic Chemistry, Vol1-5, Wiley-VCH 2011]. In both fields in the development of peptide drugs and in the synthesis of biologically active peptides there is a constant need for synthetic methods, thus numerous methods have been developed for the coupling of amino and carboxyl functional groups containing amino acids in a given sequence. First the synthesis of simple and short peptide sequences was performed by the utilization of the formation and cleavage of appropriate protecting and activating groups consecutively and in a given defined order. A major breakthrough was achieved in 1963 by the invention of Bruce Merrifield relating to the synthesis of longer sequences via the solid phase peptide synthesis (SPPS) (R. B. Merrifield: J. Am. Chem. Soc. 1963, 85, 2149-2154). This method rendered the synthesis of longer peptide chains possible only by using a high amino acid excess. Nowadays chemically prepared synthetic peptides are exclusively made by the application of this synthetic method (Norbert Sewald, Hans-Dieter Jakubke: Peptides: Chemistry and Biology, Wiley-VCH Weinheim, 2002).

The SPPS can be automatized, thus it can be used routinely. The first automated peptide synthesizer operating in batch mode was created by Merrifield (US 3,531,258). This device utilized only Boc-protecting group scheme, requiring high amino acid equivalents and long reaction times. Later on numerous chemical reactors have been developed, which are suitable for synthesis performed on solid support (US 4,746,490, US 5,288,464, US 5,380,495, US 6,028,172). In these reactors the solid support, i.e. the resin is placed in a reaction vessel, in which the solvent and dissolved reagents are transferred by a pump system or by pressurized gas. The respective reaction steps are performed in batch mode. The coupling steps are performed with the application of high excess of amino acids and reagents (5-10 equivalents) and long reaction times (120-240 min) are needed depending on the apparatus resulting in the consumption of high amounts of solvents.

The utilization of the microwave technology significantly contributed to the reduction of the reaction times (US 7,550,560). Due to this procedure the duration of the reactions could be decreased. Until today, this is the sole technique which allows the heating of the resin and the reaction mixture. Furthermore, by the application of microwave irradiation

for heating not only a thermal effect is present, but the rotation of the peptide chain backbone takes place providing a better access of the N-terminal amino group, thus a better and more efficient coupling can be achieved (STACEY A. PALASEK, ZACHARY J. COX and JONATHAN M. COLLINS: J. Pept. Sci. 2007; 13: 143-148). Nowadays, microwave assisted peptide synthesis is routinely used for the fast, automated and high purity synthesis of peptide sequences [Murray Goodman, Claudio Toniolo, Luis Moroder: Synthesis of Peptides and Peptidomimetics (Methods in Organic Chemistry) Houben-Weyl, 2002; Andrew B. Hughes: Amino Acids, Peptides and Proteins in Organic Chemistry, Vol1-5, Wiley-VCH 2011].

One of the most typical disadvantage of microwave assisted peptide synthesis technologies is on the one hand, that they require the application of high excess in amino acids (3-10 equiv.) for the completion of coupling (Norbert Sewald, Hans-Dieter Jakubke: Peptides: Chemistry and Biology, Wiley-VCH Weinheim, 2002). On the other hand, in the case of amino acids with difficult coupling properties - i.e. amino acids which do not show total coupling in case of conventional reaction circumstances - for example in the case of a synthetic process carried out in a microwave-aided automatized CEM-Liberty 1 reactor, even the manual of the reactor (http://www.cem.de/documents/pdf/produkte/peptid/app note bio-0006.pdf), furthermore methodical publications (http://www.dddmag.com/articles/2012/12/ microwave-assisted-peptide-synthesis; Bernadett Bacsa, C Oliver Kappe: Nature Protocols 2007, 2, 2222 - 2227) instruct for the utilization of double coupling. In case of double coupling, following the conventionally applied 5 or eventually even more equivalents of amino acid and coupling agents, a further 5 or more equivalents of amino acid and coupling agents are utilized in the device. Accordingly, to achieve the total coupling even the utilization of 10 to 20 equivalents of amino acids is necessary. In the case of natural $\alpha$-amino acids the arginine is a typical example for this situation (http://www.cem.de/documents/pdf/produkte/peptid/app_note_bio-0006.pdf).

The reaction time can be reduced without heating under pressure with a continuous flow methodology (US 4,192,798 patent and WO 82/03077 international patent application). To attain a higher pressure, nitrogen gas has been utilized, the typical pressures were as follows: 50 psi (3.4 bar), 200 psi (13.8 bar) and 1000 psi (68.9 bar). The flow rates varied in wide ranges, like between 10 ml/min and 20 ml/min, consequently solvent consumption of the methodology has been very high. A further disadvantage of said technology is, that during the peptide synthesis high amino acid excess is utilized, typically 4, 5, 8 or 10 equivalents, thus the synthesis of a peptide is very expensive.

Nowadays, in pharmaceutical chemistry and in material science the application of unnatural, expensive, exotic amino acids is wide-spreading due to the important use thereof. One of the most prominent application of said amino acids in material science is the utilization of lyotropic liquid crystals. Pharmaceutical activity of the peptides constructed from the unnatural, expensive, exotic amino acids is as follows: antibacterial (H-$\beta$-hPhe-$\beta$-hVal-$\beta$-hPhe-$\beta$-hVal-$\beta$-hLys-$\beta$-hVal-$\beta$-hLys-$\beta$-hVal-$\beta$-hPhe-OH), anticancer ($\beta$-hTyr-$\beta$-hGly-$\beta$-hArg-$\beta$-hLys-$\beta$-hLys-$\beta$-hArg-$\beta$-hArg-$\beta$-hGln-$\beta$-hArg-$\beta$-hArg-$\beta$-hArg-OH), cholesterol level decreasing (H-($\beta$-hAla-$\beta$-hLys-$\beta$-hPhe)$_3$-OH), $\beta$-amyloid binding ([1S,2S]-ACHC-$\beta^3$-hArg-([1S,2S]-ACHC)2-$\beta^3$-hAsp-[1S,2S]-ACHC-Gly-Gly-Cys-$NH_2$), etc. (C. M. Goodman, S. Choi, S. Shandler and W. F. DeGrado: Nat. Chem. Biol. 2007, 3, 252-262). The most prominent representatives of said amino acids are the $\beta$-amino acids and the $\beta$-peptides synthesized from $\beta$-amino acids (D. Seebach and J. Gardiner: Acc. Chem. Res. 2008, 41, 1366-1375). However, in the case of the coupling the strongly structure promoting $\beta$-amino acids double coupling is necessary also under the circumstances of the microwave assisted synthesis. It is especially true for the coupling of 2-aminocyclohexane carboxylic acid (Justin K. Murray, Bilal Farooqi, Jack D. Sadowsky, Mark Scalf, Wesley A. Freund, Lloyd M. Smith, Jiandong Chen, Samuel H. Gellman: J. AM. CHEM. SOC. 2005, 127, 13271-13280). In consequence of the required high excess and of the high prices the amino acids characterized above can not be incorporated into a peptide chain in a cost-efficient way (http://www.sigma-aldrich.com/chemistry/chemistryproducts.html?TablePage=16275558).

Thus, principal aim of the present invention was providing a method suitable to overcome the above mentioned disadvantages (high amino acid excess, the unnecessary application of high amount of solvent causing environmental pollution, high energy and material costs) and rendering thereby possible the preparation of peptides comprising natural and also expensive unnatural or strongly structure promoting amino acids.

During our investigation on solid phase peptide synthesis we surprisingly found that using the continuous flow technology to the solid support the amino acid excess can be reduced to a range of 1 to 1.5 equivalents and peptides of high purity can be synthesized with practically complete couplings and in good yields if the synthesis is performed at elevated temperature and pressure. Our invention is even more surprising, because according to the state of the art both the microwave heated and the methodologies under pressure required the application of high amino acid excess and the obtained yields were not satisfactory.

According to the present invention nearly 100% coupling can be reached even in case of using low amino acid excess.

Comparing with the previously known processes the advantage of the present invention relies not only on the necessity of remarkable low amino acid excess. Further advantages derive from the low consumption on solvent and resin during the synthesis, thus the loading of the environment can significantly be reduced. According to the mentioned advantages the realization of the method is exceptionally cheap. An additional benefit is present at the scale up of the

method i.e. not only the amounts of peptides useful for biological measurements can be synthesized, but with scale up the method is useful in the synthesis of large peptide quantities too. Nonetheless, the method can be carried out in a fast way.

## SUMMARY OF THE INVENTION

The scope of the present invention is defined by the claims. The invention is directed to a continuous flow chemical method for the solid phase synthesis of peptides at elevated temperatures and pressures.

The present invention is based on the unexpected finding that using the continuous flow technology to the solid phase peptide synthesis the amino acid excess can be reduced to a range of 1 to 1.5 equivalents and peptides of high purity can be synthesized with practically complete couplings and in good yields if the synthesis is performed at elevated temperature and pressure. Our invention is even more surprising, because according to the state of the art both the microwave heated process and the methodologies under pressure required the application of high amino acid excess and the obtained yields were not satisfactory.

According to the present invention nearly 100% coupling can be reached also when using low amino acid excess during the peptide synthesis and even in the case of peptides comprising strongly structure promoting amino acids the yields are excellent.

## DETAILED DESCRIPTION OF THE INVENTION

In one aspect the present invention provides a continuous flow chemical method for the solid phase synthesis of peptides and enantiomers, diastereomers, stereoisomers, mixtures and salts, and/or derivatives thereof, comprising the steps of:

a) preconditioning the solid support for coupling;
b) coupling an amino acid free on one terminal group and protected on the other terminal group, and optionally on any other reactive group(s), to the solid support; then
c) removing the terminal protecting group from the amino acid coupled to the solid support; then
d) coupling another amino acid free on the corresponding terminal group and protected on the other terminal group, and optionally on any other reactive group(s), to the product obtained in step c); then
repetition of the operations of steps c) and d) in succession on the protected terminal amino acid of the product obtained in each step d) as many times as necessary to obtain the desired product; and then optionally
e) after removing the terminal protecting group from the product obtained in the final step d) "capping" the product thus obtained with a compound free or activated on a reactive group and protected on any other reactive group(s); and/or
f) cleaving the peptide obtained in d) or e) steps from the solid support, and optionally converting the product obtained in step f) into a salt and/or a derivative, and/or hydrolysis thereof,

wherein the steps a)-e) are performed at a temperature ranging from 30 °C to 150 °C and at a pressure ranging from 20 bar to 500[1] bar, and in step d) the molar ratio between the product obtained in step c) and the amino acid to be coupled is not more than 1: 1.5.

In a further aspect of the invention step a) comprises the operations a1

) deprotection of the linker attached to the solid support and washing the solid support containing the free linker; or
a2) neutralization of the functional group of the linker attached to the solid support and washing the solid support; or
a3) washing the solid support containing the free linker.

According to the invention steps a)-e) are performed at the temperature range of 30 - - 150 °C, preferentially at 70 °C temperature and at the pressure range of 20 bar - 500 bar, preferentially at 60 bar pressure.

In one preferred embodiment of the invention polystyrene-polyethylene glycol copolymer or polyethylene glycol resin as solid support with (2,4-dimethoxy)benzhydrylamine linker and HATU (1-[Bis(dimethylamino)methyelen]-1H-1,2,3-triazolo[4,5-*b*]pyridinium-3-oxyde-hexafluorophosphate) or ethyl(hydroxyimino)-cyanoacetate as coupling agent can be used in organic solvents or in mixtures thereof.

Further embodiments of the invention provide a method for the coupling of all known amino acids, like natural amino acids and unnatural amino acids, hereby including $\alpha$-, $\beta$- and $\gamma$-amino acids in arbitrary order and number, preferentially 1-100 times.

Further embodiment of the invention provides in step e) a method for the synthesis of capped derivatives from the peptide attached to the resin. After cleavage these derivatives possess important application in biological investigations. For example peptides with fluorescent labelling can be synthesized for pharmacokinetic investigation or biotin labelled peptides can be synthesized for affinity matrices.

Further embodiment of the invention provides a method for the synthesis of further peptide derivatives which may be peptide acid, amide, hydrazide, ester, alcohol derivatives depending on the linker and on the cleavage step f).

Further aspects of the invention provide a method for the synthesis of peptides made of $\alpha$-, $\beta$- and $\gamma$-amino acids, including peptides with biological effect and therapeutic application, such as for example ACTH(1-24), Glucagon, Secretin, LH-RH, Cetrorelix, Leuprolide, Degarelix, Deslorelin, Triptorelin, Goserelin, Buserelin, PTH(1-34), Corticoliberin, GH-RH, GHRH(1-29), Thyroliberin, Thymosin-al, Thymopentin, Exenatide, Liraglutide; Bivalirudin; Gonadorelin; Icatibant; Sermorelin; Bortezomib; Marimastat; Pexiganan , magainin-2, IB367, Omigard; edeine D; bestatin; apstatin; amastatin; Phebestin; Probestin; microginin; and also the difficult sequences known from the literature (Norbert Sewald, Hans-Dieter Jakubke: Peptides: Chemistry and Biology, Wiley-VCH Weinheim, 2002).

Another aspect of the invention provides a method for the synthesis of cyclopeptide precursors comprising the known amino acids, including peptides with biological effect and therapeutic application, such as for example oxytocin precursor, atosiban precursor, vasopressin precursor, desmopresin precursor, Eptifibatide precursor, Pramlintide precursor, Cyclosporine A precursor; Enfuvirtide precursor, Actinomycin D precursor; Octreotide precursor, Vapreotide precursor; Octreoscan precursor, Polymyxin B1 precursor; Daptomycin precursor, Gramicidin S precursor.

The following abbreviations and definitions are used throughout the application.

Abbreviations

For the amino acids three and one letter code have been used too:

| | | |
|---|---|---|
| Alanine | Ala | A |
| Cysteine | Cys | C |
| Aspartic acid | Asp | D |
| Glutamic acid | Glu | E |
| Phenylalanine | Phe | F |
| Glycine | Gly | G |
| Histidine | His | H |
| Isoleucine | Ile | I |
| Lysine | Lys | K |
| Leucine | Leu | L |
| Methionine | Met | M |
| Asparagine | Asn | N |
| Proline | Pro | P |
| Glutamine | Gln | Q |
| Arginine | Arg | R |
| Serine | Ser | S |
| Threonine | Thr | T |
| Valine | Val | V |
| Tryptophan | Trp | W |
| Tyrosine | Tyr | Y |
| ACPC | 2-aminocyclopentane carboxylic acid | |
| ACHC | 2-aminocyclohexane carboxylic acid | |
| Pyr | L-Pyroglutamyl | |
| Fmoc | Fluorenylmethyloxycarbonyl | |
| Fmoc-GAAP | 4-((((9H-fluoren-9-yl)methoxy)carbonyl)amino)-1-acetyl-pirrolidine-2-carboxylic acid | |
| (2S,3S)-Fmoc-AHPBA | (2S,3S)-Fmoc-3-amino-2-hydroxy-4-phenyl-butanoic acid | |
| AHPA 2*S*,3*R*)-AHPA: | ($\alpha$-hydroxy-$\beta$-homophenylalanine | |
| HATU | 1-[Bis(dimethylamino)methylene]-1H-1,2,3-triazoleo[4,5-b]pyridinium 3-oxyd hexafluorophosphate | |
| DBU | 1,8-Diazabicyclo[5.4.0]undec-7-ene | |
| DMF | dimethyl-formamide | |
| DIPEA | Diisopropylethylamine | |

(continued)

| | |
|---|---|
| TFA | Trifluoroaceticacid |
| β-hAsp | β-homo-aspartic acid |
| β-hArg | β-homo-arginine |
| β-hPhe | β-homo-phenylalanine |
| β-hVal | β-homo-valine |
| β-hLys | β-homo-lysine |
| β-hTyr | β-homo-tyrosine |
| β-hGly | β-homo-glycine |
| β-hGln | β-homo-glutamine |
| β-hAla | β-homo-alaninee |
| MBHA | methyl-benzhydrylamine |
| TentaGel RRAM resin | TentaGel Research Rink amide resin. |
| Wang | hydroxymethylphenyl linker |
| SASRIN | methoxy-hydroxymethylphenyl linker |
| PAM | phenylacetamidomethyl linker |
| HMBA | hydroxymethylbenzamide linker |
| MBHA | methyl-benzhydrylamine linker |
| Rink amid | 2,4-methoxy-benzhydrylamine linker |
| Barlos | 2-chlorotritylchloride linker |
| SPPS | solid phase peptide synthesis. |

Source of chemicals used

Protected amino acids, resins, linkers, coupling and cleaving agents applied as starting material in the methods of the invention are known compounds/materials [Murray Goodman, Claudio Toniolo, Luis Moroder: Synthesis of Peptides and Peptidomimetics (Methods in Organic Chemistry) Houben-Weyl, 2002; Andrew B. Hughes: Amino Acids, Peptides and Proteins in Organic Chemistry, Vol1-5, Wiley-VCH 2011 Norbert Sewald, Hans-Dieter Jakubke Peptides: Chemistry and Biology, Wiley-VCH Weinheim, 2002], and are conventionally available [Sigma-Aldrich (H-1117 Budapest, Gábor Dénes u.2.Hungary, Chem-Impex International Inc. (935 Dillon Drive, Wood Dale, IL, 60191 USA), Rapp Polymere (Ernst-Simon-Strasse 9, 72072 Tuebingen, Germany)] or can be synthesized with known methods, for example the synthesis of Fmoc-ACPC 2-aminocyclopentane carboxylic acid is published (Forro, E.; Fulop, F. Chem. Eur. J. 2006, 12, 2587-2592); synthesis of 4-((((9H-fluoren-9-yl)methoxy)carbonyl)amino)-1-acetyl-pirrolidine-2- carboxylic acid: Fmoc-GAAP is described (Farrera-Sinfreu J, Zaccaro L, Vidal D, Salvatella X, Giralt E, Pons M, Albericio F, Royo M 2004, 126, 6048-6057); the synthesis of the TentaGel RRAM resin bound LFE-$NH_2$ starting peptide was performed by analogy of literature known method [Murray Goodman, Claudio Toniolo, Luis Moroder: Synthesis of Peptides and Peptidomimetics (Methods in Organic Chemistry) Houben-Weyl, 2002; Andrew B. Hughes: Amino Acids, Peptides and Proteins in Organic Chemistry, Vol1-5, Wiley-VCH 2011Norbert Sewald, Hans-Dieter Jakubke Peptides: Chemistry and Biology, Wiley-VCH Weinheim, 2002]; physical characteristics: ESI-MS[M+H]=408,1 m/z; HPLC RT=7,33 min (column: Phenomenex Luna C18 100A 5μm 250 x 4,6mm; eluent: 5-80% acetonitrile, 25 min).

Definitions

The term "protecting group" refers to all protecting group generally used in peptide chemistry which are suitable for use in the methods of the invention for example: protecting groups for amino groups: 9-fluorenylmethyloxycarbonyl-, tert-butyloxycarbonyl- and the like; protecting groups for carboxylic acid: tert-butyl-, cyclohexyl-, benzyl- and the like; furthermore the protecting groups of the side-chains: tert-butyl-, cyclohexyl-,trityl-, benzyloxycarbonyl-, tert-butyloxycarbonyl-, trimethylbenzenesulfonyl-, pentamethyldihydrobenzofuransulfonyl-, mesitylenesulfonyl-, tosyl-, mesyl-, [Murray Goodman, Claudio Toniolo, Luis Moroder: Synthesis of Peptides and Peptidomimetics (Methods in Organic Chemistry) Houben-Weyl, 2002; Andrew B. Hughes: Amino Acids, Peptides and Proteins in Organic Chemistry, Vol1-5, Wiley-VCH 2011].

The method of the invention can be carried out with a wide range of backbone and side-chain protecting groups, for example those cited in Murray Goodman, Claudio Toniolo, Luis Moroder: Synthesis of Peptides and Peptidomimetics (Methods in Organic Chemistry) [Houben-Weyl, 2002; Andrew B. Hughes: Amino Acids, Peptides and Proteins in Organic Chemistry, Vol. 1-5, Wiley-VCH 2011 Norbert Sewald, Hans-Dieter Jakubke Peptides: Chemistry and Biology, Wiley-VCH Weinheim, 2002] and the defined protecting group therein, for example the backbone amino group can be protected with tert-butyloxycarbonyl-, 9-fluorenylmethyloxycarbonyl groups; the backbone carboxylic acid groups can be protected with tert-butyl, benzyl, trityl groups. Preferably the 9-fluorenylmethyloxycarbonyl/tert-butyl protection technique is utilized.

The cleavage of the side-chain protecting groups can be performed in the case of tert-butyloxycarbonyl protecting group with 50-95% TFA solution, in case of 9-fluorenylmethyloxycarbonyl protecting group with a solution of 20% piperidine in DMF or with a solution of 2% DBU 2% piperidine in DMF.

The protection of the side-chain amino groups can be carried out with tert-butyloxycarbonyl-, 9-fluorenylmethyloxycarbonyl-, benzyloxycarbonyl-, trityl-groups; the protection of the side-chain carboxylic acid groups can be carried out with tert-butyl, cyclohexyl, benzyl, trityl groups; the protection of the side-chain hydroxyl groups can be carried out with tert-butyl, cyclohexyl groups; the protection of the carboxamide groups can be carried out with trityl groups; the protection of the guanidino groups can be carried out with trimethylbenzenesulfonyl, pentamethyldihydrobenzofuransulfonyl, mesitylenesulfonyl groups; preferentially the protection of the amine group can be carried out with tert-butyloxycarbonyl, 9-fluorenylmethyloxycarbonyl, benzyloxycarbonyl and trityl groups; protection of the carboxylic acid group can be carried out with tert-butyl and cyclohexyl groups; protection of the hydroxyl group can be carried out with tert-butyl-groups; protection of the carboxamide group can be carried out with trityl groups; the protection of the guanidino groups can be carried out with pentamethyldihydrobenzofuransulfonyl group.

The tert-butyloxycarbonyl, trityl, tert-butyl, pentamethyldihydrobenzofuransulfonyl protecting groups can be removed with 90-95% TFA solution. The benzyloxycarbonyl, cyclohexyl, benzyl, trimethylbenzenesulfonyl, mesitylenesulfonyl groups can be removed with HF. The 9-fluorenylmethyloxycarbonyl protecting group can be removed with a solution of 20% piperidine in DMF or with a solution of 2% DBU 2% piperidine in DMF.

The method according to the invention can be performed with all known linkers, for example: chloromethylphenyl, Wang (hydroxymethylphenyl), SASRIN (methoxy-hydroxymethylphenyl), PAM (phenyl-acetamidomethyl), HMBA (hydroxymethylbenzamide), MBHA (methylbenzhydrylamine), Rink amide (2,4-dimethoxybenzhydrylamine), Barlos (2-chlorotritylchloride) and the like; preferentially the synthesis is carried out with Rink amide linker [Murray Goodman, Claudio Toniolo, Luis Moroder: Synthesis of Peptides and Peptidomimetics (Methods in Organic Chemistry) Houben-Weyl, 2002; Andrew B. Hughes: Amino Acids, Peptides and Proteins in Organic Chemistry, Vol1-5, Wiley-VCH 2011].

The method of the invention can be performed on wide range of solid supports and resins. For example the method of the invention can be carried out on polystyrene, polystyrene-polyethylene glycol copolymer, polyamide resins and the like; the method is carried out preferentially on Tentagel RRAM polystyrene-polyethylene glycol copolymer or on Chemmatrix polyethylene glycol resin [Murray Goodman, Claudio Toniolo, Luis Moroder: Synthesis of Peptides and Peptidomimetics (Methods in Organic Chemistry) Houben-Weyl, 2002; Andrew B. Hughes: Amino Acids, Peptides and Proteins in Organic Chemistry, Vol1-5, Wiley-VCH 2011].

The method according to the invention can be performed in organic solvents, mixtures and admixtures thereof, including the multiphase systems, ionic liquids (Alesia A. Tietze, Pascal Heimer, Annegret Stark, Diana Imhof: Molecules 2012, 17, 4158 8-4185).

The suitable organic solvents can be non-polar or polar or mixture thereof or multiphase mixture thereof. Preferentially as adequate organic solvents halogenated hydrocarbons, like methylene-dichloride, dichloroethane, chlorobenze, etc.; ketone type solvents like acetone, butan-2-on, acetophenone, etc.; hydrocarbons like toluene, hexane, heptane, etc.; ethers like diethyl ether, tetrahydrofurane, tert-butyl-methyl ether, or further polar solvents as nitriles like acetonitrile, etc; amides like N,N-dimethyl-formamide, N,N-dimethyl-acetamide, N-methyl-pyrrolidon, etc.; amines like N,N-diizopropyl-ethylamine, etc. and the like may be used. Preferentially N,N-dimethyl-formamide, chloroform, N-methyl-pyrrolidon are used as solvent.

The method according to the invention is suitable for the coupling of all known amino acids in any order and number, examples for natural amino acids: A, C, D, E, F, G, H, I, K, L, M, N, P, Q, R, S, T, V, W, Y, and for examples for non-natural amino acids, including α-, β- and γ-amino acids, D-naphthylalanine, D-4-chlorophenylalanine, D-(3'-pyridyl)-alanine-D-citrulline-D-alanine, D-Leu, Pyroglutamine, S-iso-propylaminoglycine, D-4-carbamidophenylalanine, L-(4-(2,6-dioxohexahydropyrimidine-4-carboxamido)-phenylalanine, D-naphthylalanine, D-Trp, D-Ser(OtBu), D-Arg; hydroxyproline, thiophenylalanine, (S)-1,2,3,4-tetrahydroisoquinolin-3-carboxylic acid, (3aS,7aS)-octahydro-1H-indole-2-carboxylic acid, (R)-(1-amino-3-methylbutyl)boronic acid, S-tert-butylglycine, (2*S*,3*R*)-AHPA, (2*S*,3*R*)-α-hydroxy-β-homoleucine, 3-amino-2-hydroxydecanoic acid, NMeTyr, desamino-cystein, D-Tyr(Et), homoarginine, (2*S*,3*R*,4*R*,6*E*)-3-hydroxy-4-methyl-2-(methylamino)okt-6-ene carboxylic acid, S-ethylglycine, *N*-methylglycine, *N*-methyl-L-leucine, D-alanine, *N*-methyl-L-valine, NMeGly, L-ornithine, 3-anthranyloyl-L-alanine, β-hydroxyglutamic acid, aminoglycine, ACPC, ACHC, (2S,3S)-Fmoc-AHPBA, Fmoc-GAAP [Murray Goodman, Claudio Toniolo, Luis Moroder: Synthesis of Peptides and Peptidomimetics (Methods in Organic Chemistry) Houben-Weyl, 2002; Andrew B. Hughes: Amino Acids, Peptides and Proteins in Organic Chemistry, Vol1-5, Wiley-VCH 2011].

The method according to the invention can be performed with different coupling agents or with the mixtures thereof [Murray Goodman, Claudio Toniolo, Luis Moroder: Synthesis of Peptides and Peptidomimetics (Methods in Organic Chemistry) Houben-Weyl: Vol. E 22 a-d 2002; Andrew B. Hughes: Amino Acids, Peptides and Proteins in

Organic Chemistry, Vol1-5, Wiley-VCH 2011]. According to a preferred embodiment the following coupling agents are appropriate: mixed anhydride forming agents: ethylchloroformate, isobutylchloroformate; symmetrical anhydride forming agents: phosgene; carbodiimides: dicyclohexyl-carbodiimide, diisopropyl-carbodiimide, carbonyl-diimidazol, N-carboxy anhydrides; active ester forming agents: hydroxysuccinimide, hydroxybenzotriazole, N- -hydroxy-5-norbornene-2,3-dicarboxymide, 1-hydroxy-7-azabenzotriazole, pentafluorophenol; amino acid fluoride forming agents: tetramethyl-fluoroformamidinium hexafluorophosphate, 2-diethylamino-sulfur-trifluoride; amino acid chloride forming agents: tetramethylchloroformamidinium hexafluorophosphate, 2-chloro-1,3-dimethyl-imidazolidinium hexafluorophosphate; phosphonium reagents: benzotriazole-1-yloxytris(dimethylamino)phosphonium hexafluorophosphate, benzotriazole-1-yloxytripyrrolidinophosphonium hexafluorophosphate, azabenzotriazole-1-yloxytripyrrolidinophosphonium hexafluorophosphate, bromo-tripyrrolidinophosphonium hexafluorophosphate, 3 -(diethoxyphosphoryloxy)-1,2,3 -benzotriazine-4(3 H)-one; uronium reagents: N,NN',N'--tetramethyl-O-(1H-benzotriazole-1-yl)uronium hexafluorophosphate, (1-[bis(dimethylamino)-methylene] -1H-1,2,3-triazolo[4,5-*b*]pyridinium 3-oxyde hexafluorophosphate), N,N,N',N-tetramethyl-O-(1H-benzotriazole-1-yl)uronium-tetrafluorborate, 1-[(dimethylamino)-(dimethyliminium)-methyl]-1H-1,2,3-triazolo[4,5-b]pyridine-3-oxyde hexafluorophosphate, O-(azabenzotriazole-1-yl)-1,1 ,3,3 -tetramethyleneuronium-hexafluorophosphate, O-(azabenzotriazole-1-yl)-1,1,3,3-pentamethyleneuronium hexafluorophosphate, O-(7-azabenzotriazole-1-yl)-1,3 -dimethyl- ,3-dimethyleneuronium; oxyma reagents: ethyl-(hydroxyimino)cianoacetate, (1-ciano-2-etoxy-2-oxoethylideneaminooxy)dimethylamino-morfolino-carbenium hexafluorophosphate, O-[(etoxycarbonyl)cianomethyleneamino]-N,N,N',N'-tetramethylluronium-hexafluorophosphate. More preferred coupling agents are for example: 1-[bis(dimethylamino)methylene]-1H-1,2,3-triazolo[4,5-*b*]pyridinium-3-oxyde-hexafluorophosphate and ethyl-(hydroxyimino)-cianoacetate.

The deprotection step a1) is performed at elevated temperature and pressure with any known methods, for example: in the case of polystyrene, polystyrene-polyethylene glycol copolymer, polyethylene glycol, polyamide resin with Rink amide ((2,4-dimethoxy)benzhydrylamine) linker the deprotection can be performed with known deprotecting agents [Murray Goodman, Claudio Toniolo, Luis Moroder: Synthesis of Peptides and Peptidomimetics (Methods in Organic Chemistry) Houben-Weyl: Vol. E 22 a-d 2002; Andrew B. Hughes: Amino Acids, Peptides and Proteins in Organic Chemistry, Vol1-5, Wiley-VCH 2011], preferentially with a solution of 2% DBU and 2% piperidine in an appropriate solvent, preferentially in DMF solution. The following washing step is practically performed with the previously applied solvent. Or

The neutralization step a2) is performed at elevated temperature and pressure with any known methods, for example in the case of polystyrene, polystyrene-polyethylene glycol copolymer, polyethylene glycol or polyamide resin with MBHA (methylbenzhydrylamine) linker known bases can be used, preferentially triethylamine in an appropriate solvent, preferentially using a solution in DMF. The following washing step is practically performed with the previously applied solvent.

The washing step a3) is performed at elevated temperature and pressure with all known solvents, preferentially with the previously used solvent, for example in the case of polystyrene, polystyrene-polyethylene glycol copolymer, polyethylene glycol or polyamide resin with chloromethylphenyl, Wang (hydroxymethylphenyl), SASRIN (methoxy-hydroxymethylphenyl), PAM (phenylacetamidomethyl), HMBA (hydroxymethylbenzamide), Barlos (2-chlorotritylchloride) linker the washing step is performed with DMF.

The coupling step b) is performed at elevated temperature and pressure for example in the case of polystyrene, polystyrene-polyethylene glycol copolymer, polyethylene glycol, polyamide resin with chloromethylphenyl, Wang (hydroxymethylphenyl), SASRIN (methoxy-hydroxymethylphenyl), PAM (phenylacetamidomethyl), HMBA (hydroxymethylbenzamide), MBHA (methylbenzhydrylamine), Rink amide ((2,4-dimethoxy)benzhydrylamine), Barlos (2-chlorotritylchloride) linker with known coupling agents [Murray Goodman, Claudio Toniolo, Luis Moroder: Synthesis of Peptides and Peptidomimetics (Methods in Organic Chemistry) Houben-Weyl: Vol. E 22 a-d 2002; Andrew B. Hughes: Amino Acids, Peptides and Proteins in Organic Chemistry, Voll-5, Wiley-VCH 2011], preferentially with HATU in a given solvent, preferentially in DMF. The following washing step is practically performed with the previously applied solvent.

The deprotection step c) is performed at elevated temperature and pressure with known deprotecting agent, preferentially with DBU and piperidine in a given solvent, preferentially in DMF. The following washing step is practically performed with the previously applied solvent.

The coupling step d) is performed at elevated temperature and pressure with known coupling agents, preferentially with HATU in a given solvent, preferentially in DMF. The following washing step is practically performed with the previously applied solvent.

According to the method of invention the operations of steps c) and d) are repeated in succession on the protected terminal amino acid of the product obtained in each step d) as many times as necessary for obtaining the desired product.

Optionally in capping step e) of the method of the invention fluorescein, biotin acyl group capped peptide derivatives can be produced [Murray Goodman, Claudio Toniolo, Luis Moroder: Synthesis of Peptides and Peptidomimetics (Methods in Organic Chemistry) Houben-Weyl, 2002; Andrew B. Hughes: Amino Acids, Peptides and Proteins

in Organic Chemistry, Vol1-5, Wiley-VCH 2011Norbert Sewald, Hans-Dieter Jakubke Peptides: Chemistry and Biology, Wiley-VCH Weinheim, 2002].

The capping step e) is performed at elevated temperature and pressure for example in the case of polystyrene, polystyrene-polyethylene glycol copolymer, polyethylene glycol, polyamide resin with a peptide bound to a linker as for example chloromethylphenyl, Wang (hydroxymethylphenyl), SASRIN (methoxy-hydroxymethylphenyl), PAM (phenylacetamidomethyl), HMBA (hydroxymethylbenzamide), MBHA (methylbenzhydrylamine), Rink amide ((2,4-dimethoxy)benzhydrylamine), Barlos (2-chlorotritylchloride). Wherein after deprotection of the terminal functional group of the peptide produced in the last step d) it is reacted with a compound having one reactive group free and protected on further reactive group(s) in the presence of known coupling agents [Murray Goodman, Claudio Toniolo, Luis Moroder: Synthesis of Peptides and Peptidomimetics (Methods in Organic Chemistry) Houben-Weyl: Vol. E 22 a-d 2002; Andrew B. Hughes: Amino Acids, Peptides and Proteins in Organic Chemistry, Vol1-5, Wiley-VCH 2011], preferentially applying HATU in a given solvent, preferentially in DMF or applying a reagent bearing an activating group, but protected on further reactive group(s) preferentially in DMF as solvent. The following washing step is practically performed with the previously applied solvent.

According to the method of the invention peptides in form of acid, amide, hydrazide, thiol, ester, alcohol derivatives can be produced depending on the cleavage conditions from the resin and the linker. In case of 2-chlorotritylchloride linker the cleavage step will result in the formation of a peptide acid derivative when hydrogen fluoride or trifluoromethanesulfonic acid is used. In case of Wang (hydroxymethylphenyl) linker the cleavage step will result in the formation of a peptide acid derivative when TFA is used. In case of SASRIN (methoxy-hydroxymethylphenyl) linker the cleavage step will result in the formation of a peptide acid derivative with the application of TFA. In case of PAM (phenylacetamidomethyl) linker the cleavage step will result in the formation of a peptide acid derivative with the application of hydrogen fluoride or trifluoromethanesulfonic acid. In case of MBHA (methylbenzhydrylamine) linker the cleavage step will result in the formation of a peptide amide derivative with the application of hydrogen fluoride or trifluoromethanesulfonic acid. In case of Rink amide ((2,4-dimethoxy)benzhydrylamine) linker the cleavage step will result in the formation of a peptide amide derivative with the application of TFA. In case of Barlos (2-chlorotritylchloride) linker the cleavage step will result in the formation of a peptide acid derivative with the application of TFA. In case of HMBA (hydroxymethylbenzamide) linker the cleavage step will result in the formation of a peptide amide derivative with the application of methanolic solution of ammonia or primary and secondary amine, the cleavage step will result in the formation of hydrazide derivative with the application of hydrazine, the cleavage step will result in the formation of ester derivatives with the application of alcohols, the cleavage step will result in the formation of peptide alcohols with the application of lithium borohydride [Murray Goodman, Claudio Toniolo, Luis Moroder: Synthesis of Peptides and Peptidomimetics (Methods in Organic Chemistry) Houben-Weyl, 2002; Andrew B. Hughes: Amino Acids, Peptides and Proteins in Organic Chemistry, Vol1-5, Wiley-VCH 2011Norbert Sewald, Hans-Dieter Jakubke Peptides: Chemistry and Biology, Wiley-VCH Weinheim, 2002].

The cleavage step f) can be performed with all known cleaving agents, preferentially with a solution of TFA, which contains 5-10% of water and 2.5-5% of scavenging agent, which is preferentially triisopropylsilane and dithiothreitol.

Optionally salts and further derivatives can be produced from the peptides cleaved from the resin cleaved peptides by generally known methods. [Murray Goodman, Claudio Toniolo, Luis Moroder: Synthesis of Peptides and Peptidomimetics (Methods in Organic Chemistry) Houben-Weyl, 2002; Andrew B. Hughes: Amino Acids, Peptides and Proteins in Organic Chemistry, Vol1-5, Wiley-VCH 2011Norbert Sewald, Hans-Dieter Jakubke Peptides: Chemistry and Biology, Wiley-VCH Weinheim, 2002].

Preferred salts of the peptides synthesized by the method of the invention are the pharmaceutically acceptable salts, which are known per se as non-toxic salts, for example acid addition salts, salts formed by organic acids, (e.g. acetate, trifluoroacetate, maleate, tartarate, fumarate, methanesulfonate, benzenesulfonate, formiate, toluenesulfonate, and the like), salts formed by inorganic acids (e.g. hydrochloride, hydrobromide, hydroiodide, sulfate, nitrate, phosphate and the like), furthermore salts formed with bases, like salts formed with amino acids (e.g. with arginine, aspartic acid, glutamic acid and the like), salts formed with alkali metals (e.g. sodium salt, potassium salt and the like), with alkaline earth metals (calcium salt, magnesium salt and the like), the ammonium salts, furthermore salts formed with organic bases (e.g. trimethylamine, triethylamine, pyridine, picoline, dicyclohexylamine, N,N-dibenzyl-ethylene-diamine and the like) and the like.

The method of invention can be performed in continuous flow devices equipped with a tool for tuning the flow rate, temperature and pressure, an apparatus which is able to work according the parameters described in the description. For example the devices of the following producers are suitable: Thalesnano Inc., http://www.thalesnano.com/; Future Chemstry Holding BV., http://www.futurechemistry.com/; Syrris Ltd. http://www.syrris.com/; Uniqsis Ltd. http://www.uniqsis.com/; Chemtrix BV http://www.chemtrix.com/flow-chemistry

The flow rate can be varied depending on the device in the range of 0.01 - 10 ml/min, preferentially it is 0.1 ml/min.

Methods and apparatus used in the analytics of the synthesized compounds: HPLC-MS: Dionex-Thermo LCQ Fleet, Phenomenex Luna C18 5um 100A 4.6*250mm; A eluent 0.1% acetic acid in water, B eluent 0.1% acetic acid in acetonitrile.

The coupling efficiency can be calculated as follows:
The coupling efficiency value was determined with the application of HPLC-MS apparatus: Phenomenex Luna C18 5um 100A 4.6*250mm; gradient: 5% B -> 80% B 25 min; 1.2 ml/min.

The R and P are determined from area under the curve values on the HPLC-MS ion chromatogram, where R relates the non reacted reactant starting material, while P refers to the product. From these values the coupling efficiency can be calculated as follows:

$$C(\%)=[P/(R+P)]*100$$

The coupling efficiency value is the most important parameter to describe the efficacy of peptide synthesis, because during a multistep method, in each coupling step side products hindering the purification are generated as a result of a not satisfactory coupling, and the amount of side products multiplies with the number of steps. For example, if each coupling step is performed with 99% coupling efficiency, than after the $10^{th}$ step the purity of the product is maximally 90%. If 100 steps are required, than calculating with the previous parameters the purity of the product is maximally 37%. However, if the coupling efficiency is 95%, after 10 steps the purity of the product is maximally 60%, while after 100 steps the purity of the product is maximally 0.6% (Norbert Sewald, Hans-Dieter Jakubke: Peptides: Chemistry and Biology, Wiley-VCH Weinheim, 2002 pp. 229). Thus few percent increase in coupling efficiency provides significant increase in the final yield.

The determination of the yield has been made by methods known from the literature.

The loading of the resin means the value, which shows, that how many mmol of amino groups are on 1 gram of the resin. This value is determined by the producer.

**EXAMPLES**

The following examples are only for purposes of illustration of the invention.

General procedures

Step a) preconditioning the solid support for coupling
Step a1) deprotection of the linker attached to the solid support and washing the solid support containing the free linker
The protecting group was removed from the Rink amide (2,4-dimetoxy-benzhydrylamine) linker of the previously swollen polystyrene, polystyrene-polyethylene glycol copolymer, polyethylene glycol, polyamide resin at 60 bar pressure and at 70 °C temperature with a solution of 2% DBU and 2% piperidine in DMF; afterward the resin containing free linker group was washed at 60 bar pressure and at 70 °C temperature; or
Step a2) neutralization of the functional group of the linker attached to the solid support and washing the solid support
The linker group of the previously swollen polystyrene, polystyrene-polyethylene glycol copolymer, polyethylene glycol, polyamide resin equipped with a methyl-benzhydrylamine linker was neutralized with 10% triethylamine solution in DMF at 60 bar pressure and at 70 °C temperature; or
Step a3) washing the solid support containing the free linker
The free linker group containing polystyrene, polystyrene-polyethylene glycol copolymer, polyethylene glycol, polyamide resin equipped with a with chloromethylphenyl, Wang (hydroxymthylphenyl), SASRIN (methoxy-hydroxymthylphenyl), PAM (phenylacetamidomethyl), HMBA (hydroxymthylbenzamide), Barlos (chlorotriphenylmethyl-chloride) linker was washed at 60 bar pressure and at 70 °C temperature.
Step b) coupling an amino acid free on one terminal group and protected on the other terminal group, and optionally on any other reactive group(s), to the solid support
An amino acid free on one terminal group and protected on the other terminal group, and optionally on any other reactive group(s), was coupled through the linker group to the solid support with HATU as coupling agent at 60 bar pressure and at 70 °C temperature; afterward the solid support was washed at 60 bar pressure and at 70 °C temperature;
Step c) removing the terminal protecting group from the amino acid coupled to the solid support
The terminal protecting group was removed with a solution of 2% DBU and 2% piperidine in DMF from the amino acid coupled to the solid support in the previous step b) at 60 bar pressure and at 70 °C temperature; afterward resin was washed at 60 bar pressure and at 70 °C temperature;
Step d) coupling another amino acid free on the corresponding terminal group and protected on the other terminal group, and optionally on any other reactive group(s), to the product obtained in step c)

Another amino acid free on the corresponding terminal group and protected on the other terminal group, and optionally on any other reactive group(s) was coupled with HATU as coupling agent to the product obtained in step c) at 60 bar pressure and at 70 °C temperature, afterward resin was washed at 60 bar pressure and at 70 °C temperature; the operations of steps c) and d) were repeated in succession on the protected terminal amino acid of the product obtained in each step d) as many times as necessary to obtain the desired product; and then optionally

Step e) after removing the terminal protecting group from the product obtained in the final step d) "capping" the product thus obtained with a compound free or activated on a reactive group and protected on any other reactive group(s)

After the removal of the terminal protecting group from the product obtained in the last step d) at 60 bar pressure and at 70 °C temperature the deprotected product was capped with known hydroxysuccinimide esters or in case of free carboxylic acid functional group with known coupling agents e.g. with HATU or with carboxylic acid derivatives e.g. with carboxylic acid chlorides, carboxylic acid anhydrides, which was followed by washing;

Step f) cleaving the peptide obtained in steps d) or e) steps from the solid support, and optionally converting the product obtained in step f) into a salt and/or a derivative, and/or hydrolysis thereof

The peptide was cleaved at room temperature with a solution of 90% TFA, which preferentially contains 5-10% of water and 2.5-5% of a scavenging agent, which is preferentially triisopropylsilane and dithiothreitol. In case of Wang (hydroxymthylphenyl) linker peptide acids and in case of Rink amide (2,4-dimetoxy-benzhydrylamine) linker peptide amides are obtained under these conditions.

Optionally during the step f) or afterward the obtained product was converted into salts and/or derivatives and/or was hydrolyzed with known methods.

General method for peptide synthesis:

The synthesis was performed on 0.03 mmol scale. After swelling of the resin (0.03 mmol) in DMF it was filled into the peptide synthesis reactor. Then the reactor was connected into the flow line, wherein the flow rate was adjusted to 0.1 ml/min, and the pressure was set to 60 bar and the temperature to 70 °C. For the removal of protecting group from the amino group of the linker located on the TentaGel Research Rink amide resin, 0.5 ml of a solution of 2% DBU, 2% piperidine in DMF was applied for 5 min in continuous flow. It was followed by a washing, where DMF was pumped for 3 min through the peptide synthesis reactor. For the coupling step 0.045 mmol Fmoc protected amino acid, 0.045 mmol HATU coupling agent and 0.09 mmol DIPEA base dissolved in 1 ml DMF were applied. This solution was recirculated in the reactor for 15 min. Then the reactor was washed with DMF during 3 min which was followed by a deprotection according to step c) where 0.5 ml of a solution of 2% DBU, 2% piperidine in DMF was applied for 5 min in continuous flow. Then the reactor was washed with DMF during 3 min. For the next coupling step d) 0.045 mmol Fmoc protected amino acid, 0.045 mmol HATU coupling agent and 0.09 mmol DIPEA base were dissolved in 1 ml DMF. This solution is recirculated in the reactor for 15 min. Finally a washing with DMF was effectuated. With the repetition of the operations of steps c) and d) in succession on the protected terminal amino acid of the product obtained in each step d) as many times as necessary to obtain the desired product the desired length of the peptide was reached.

After the last step d) optionally in a capping step e) a fluorescein labeled product was prepared from the peptide obtained in last step d) and deprotected according to step c) by applying 0.045 mmol carboxyfluorescein, 0.045 mmol HATU coupling agent and 0.09 mmol DIPEA base 1 ml DMF solution , or an acyl derivative was prepared by appliying 0.045 mmol acetic anhydride and 0.09 mmol DIPEA base in 1 ml DMF solution. Each solution was recirculated in the reactor for 15 min respectively. Then the reactor was washed as above.

Before the cleavage step f) the column in the reactor was cooled down to room temperature and a solution of 2 ml TFA, water, triisopropylsilane and dithiothreitol (90:5:2.5:2.5) was applied at room temperature for 2 hours at atmospheric pressure with recirculation.

For the coupling, washing, deprotection of one amino acid we utilized 2.1 ml DMF altogether.

The raw peptides were characterized by the HPLC-MS data.

**Comparative examples 1-4**

Synthesis of the ALFE-$NH_2$ peptide at different temperatures and at atmospheric pressure

Synthesis of the ALFE-$NH_2$ peptide from the resin bound LFE peptide as starting material at different temperatures and at atmospheric pressure by the application of 1.5 equivalents of amino acid.

According to the general coupling step d) the coupling of 14 mg (0.045 mmol) Fmoc-Ala-OH was performed in 0.03 mmol scale to the TentaGel RRAM resin bound LFE tripeptide by the utilization of 17 mg (0.045 mmol) HATU and 16 uL (0.09 mmol) DIPEA at atmospheric pressure at different temperatures with 0.1 ml/min flow rate.

**Table 1.** Coupling efficiency at different temperature values at the synthesis ALFE-$NH_2$ peptide

| Example | Temperature (°C) | Coupling efficiency (C%) |
|---|---|---|
| 1 (comparative) | 30 | 68 |
| 2 (comparative) | 50 | 72 |
| 3 (comparative) | 70 | 88 |
| 4 (comparative) | 90 | 86 |

The results show, that at the coupling of one Fmoc-Ala-OH which is an easily coupling amino acid, in case of 1.5 amino acid equivalent at 90 °C nearly 14% of the uncoupled product remains.

**Examples 6-9**

Synthesis of the ALFE-$NH_2$ peptide at different pressure values and at 70 °C temperature

Synthesis of the ALFE-$NH_2$ peptide from the resin bound LFE peptide as starting material at different pressure values and at 70 °C temperature by the application of 1.5 equivalents of amino acid.

According to the general coupling step d) the coupling of 14 mg (0.045 mmol) Fmoc-Ala-OH was performed in 0.03 mmol scale to the TentaGel RRAM resin bound LFE tripeptide by the utilization of 17 mg (0.045 mmol) HATU and 16 uL (0.09 mmol) DIPEA at different pressure values at 70 °C temperatures with 0.1 ml/min flow rate.

**Table 2.** Coupling efficiency on different pressure values at 70 °C temperature by the synthesis of the ALFE-$NH_2$ peptide

| Example | Temperature (°C) | Pressure (bar) | Coupling efficiency (C %) |
|---|---|---|---|
| 3 (Comparative) | 70 | 1 | 88 |
| 6 | 70 | 20 | 92 |
| 7 | 70 | 40 | 97 |
| 8 | 70 | 60 | >99 |
| 9 | 70 | 80 | >99 |

The results show, that an essential total coupling can be reached with 1.5 equivalent of Fmoc-Ala-OH at elevated temperature and pressure.

**Examples 10-12**

Synthesis of the ALFE-$NH_2$ peptide at 60 bar pressure and at 70 °C temperature by the application of different amino acid equivalents

According to the general coupling step d) the coupling of 14 mg (0.045 mmol), or 11 mg (0,036 mmol), or 9 mg (0,03 mmol) Fmoc-Ala-OH was performed in 0.03 mmol scale to the TentaGel RRAM resin bound LFE tripeptide by the utilization of 17 mg (0.045 mmol), or 14 mg (0,036 mmol) or 11 mg (0,03 mmol) HATU and 16 $\mu$L (0.09 mmol), or 13 $\mu$L (0,072 mmol), or 11 $\mu$L (0,06 mmol) DIPEA at 60 bar pressure and at 70 °C temperature with 0.1 ml/min flow rate.

**Table 3.** Coupling efficiency at 60 bar pressure and at 70 °C temperature in case of different amino acid equivalents at the synthesis ALFE-$NH_2$ peptide

| Example | Amino acid equivalents | Coupling efficiency (C %) |
|---|---|---|
| 10 | 1.5 | >99 |
| 11 | 1.2 | 97 |
| 12 | 1 | 94 |

**Method for the coupling of natural α-amino acids**

**Examples 13-32**

Coupling of natural α-amino acids to the resin bound LFE tripeptide at 60 bar pressure and at 70 °C temperature by the application of 1.5 amino acid equivalent.

According to the general coupling step d) couplings of natural α-amino acids are performed in 0.03 mmol scale to the TentaGel RRAM resin bound LFE tripeptide by the utilization of 17 mg (0.045 mmol) HATU and 16 μL (0.09 mmol) DIPEA at 60 bar pressure at 70 °C temperatures with 0.1 ml/min flow rate.

**Table 4.** Coupling of natural α-amino acids with 1.5 equivalents on the resin bound LFE tripeptide at 60 bar pressure at 70 °C temperature.

| Example | Amino acid | Product | Coupling efficiency (C%) |
|---|---|---|---|
| 13 | Fmoc-Ala-OH | ALFE-$NH_2$ | >99 |
| 14 | Fmoc-Arg(Pbf)-OH | RLFE-$NH_2$ | >99 |
| 15 | Fmoc-Asp(OtBu)-OH | DLFE-$NH_2$ | 99 |
| 16 | Fmoc-Asn(Trt)-OH | NLFE-$NH_2$ | >99 |
| 17 | Fmoc-Tyr(tBu)-OH | YLFE-$NH_2$ | >99 |
| 18 | Fmoc-Thr(tBu)-OH | TLFE-$NH_2$ | >99 |
| 19 | Fmoc-Phe-OH | FLFE-$NH_2$ | >99 |
| 20 | Fmoc-Pro-OH | PLFE-$NH_2$ | >99 |
| 21 | Fmoc-Gln(Trt)-OH | QLFE-$NH_2$ | >99 |
| 22 | Fmoc-Met-OH | MLFE-$NH_2$ | >99 |
| Example | Amino acid | Product | Coupling efficiency (C %) |
| 23 | Fmoc-Glu(OtBu)-OH | ELFE-$NH_2$ | >99 |
| 24 | Fmoc-Lys(Boc)-OH | KLFE-$NH_2$ | >99 |
| 25 | Fmoc-Val-OH | VLFE-$NH_2$ | 99 |
| 26 | Fmoc-Gly-OH | GLFE-$NH_2$ | >99 |
| 27 | Fmoc-Cys(Trt)-OH | CLFE-$NH_2$ | 99 |
| 28 | Fmoc-Ile-OH | ILFE-$NH_2$ | 99 |
| 29 | Fmoc-Leu-OH | LLFE-$NH_2$ | >99 |
| 30 | Fmoc-Ser(tBu)-OH | SLFE-$NH_2$ | >99 |
| 31 | Fmoc-Trp(Boc)-OH | WLFE-$NH_2$ | >99 |
| 32 | Fmoc-His(Trt)-OH | HLFE-$NH_2$ | >99 |

The results indicate, that in the step d) of the invention the coupling of each amino acid is practically complete.

**Method for the synthesis of difficult sequences**

**Examples 33-34**

Synthesis of a difficult sequence: acyl carrier protein 65-74 (ACP 65-74) fragment VQAAIDYING-$NH_2$

The acyl carrier protein 65-74 (ACP 65-74) fragment is a difficult sequence and the key step of synthesis of the bioactive acyl carrier protein (LES P. MIRANDA AND PAUL F. ALEWOOD Proc. Natl. Acad. Sci. USA 1999, 96, 1181-1186). The synthesis of the peptide was performed in two different ways. In the first case the synthesis was performed with method of the present invention in a continuous flow reactor with 1.5 amino acids equivalents atn 60 bar pressure at 70 °C temperature (flow rate 0.1 ml/min). In the second case the synthesis was performed on the same resin, on the same temperature, with the same coupling agent, with the same amino acids, with the same amino acid equivalents in a microwave reactor. The total yield of the 10 couplings with the method of the present invention is 86%.

**Table 5.** Synthesis of the difficult sequence Acyl carrier protein 65-74 (ACP 65-74) fragment

| Example | Method | Temperature (°C) | Amino acid equivalents | Coupling efficiency (C%) |
|---|---|---|---|---|
| 33 | Method of the invention (60 bar pressure) | 70 | 1.5 | 86 |
| 34 (comparative) | Microwave (without pressure) | 70 | 1.5 | 62 |

**Examples 35-36**

Synthesis of the GILTVSVAV-$NH_2$ difficult sequence

The GILTVSVAV-$NH_2$ difficult sequence is a fragment of a CD8+ T-cell epitope protein, the key element of synthesis thereof is the GILTVSVAV-$NH_2$ difficult sequence (Bernadett Bacsa, Kata Horváti, Szilvia Bősze, Fritz Andreae, C. Oliver Kappe Journal of Organic Chemistry, 2008, 73, 7532-7542).

The synthesis of the peptide was performed in two different ways. In the first case the synthesis was performed with method of the present invention in a continuous flow reactor on Chemmatrix resin with 1.5 amino acids equivalents on 60 bar pressure on 70 °C temperature (flow rate 0.1 ml/min). In the second case the synthesis was performed on the same resin, on the same temperature, with the same coupling agent, with the same amino acids, with the same amino acid equivalents in a microwave reactor. The total yield of the 9 couplings with the method of the present invention is 86%.

**Table 6.** The synthesis of difficult sequence GILTVSVAV-$NH_2$

| Example | Method | Temperature (°C) | Amino acid equivalents | Coupling efficiency (C %) |
|---|---|---|---|---|
| 35 | Method of the invention (60 bar pressure) | 70 | 1.5 | 86 |
| 36 (comparative) | Microwave (without pressure) | 70 | 1.5 | 39 |

**Method for the synthesis of β-peptides**

β-Peptides form part of compounds with biological activity, their synthesis plays a key element in the development of future drugs. An important pharmacological property of ACPC containing peptides is the antibacterial effect (Richard P. Cheng, Samuel H. Gellman, William F. DeGrado Chem. Rev. 2001, 101, 3219-3232).

**Examples 37-38**

Synthesis of cis-2-aminocyclopentanecarboxylic acid hexamer with alternating chirality H-([1S,2R]-ACPC-[1R,2S]-ACPC)$_3$-$NH_2$

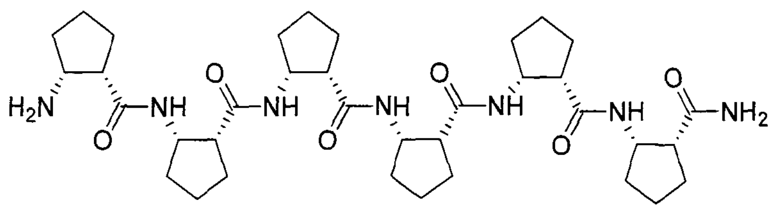

The synthesis of the peptide have been performed in two different ways. In the first case the synthesis was performed on Tentagel RRAM resin with method of the present invention in a continuous flow reactor with 1.5 amino acids equivalents at 60 bar pressure and at 70 °C temperature (flow rate 0.1 ml/min). In the second case the synthesis was performed on the same resin, on the same temperature, with the same coupling agent, with the same amino acids, with the same amino acid equivalents in a microwave reactor. The total yield of the 6 couplings with the method of the present invention is 96%.

**Table 7.** Synthesis of *cis*-2-aminocyclopentanecarboxylic acid hexamer with alternating chirality

| Example | Method | Temperature (°C) | Amino acid equivalents | Coupling efficiency (C%) |
|---|---|---|---|---|
| 37 | Method of the invention (60 bar pressure) | 70 | 1.5 | 96 |
| 38 (comparative) | Microwave (without pressure) | 70 | 1.5 | 47 |

**Examples 39-40**

Synthesis of *cis*-2-aminocyclopentanecarboxylic acid hexamer with monotonous chirality H-([1R,2S]-ACPC)$_6$-NH$_2$

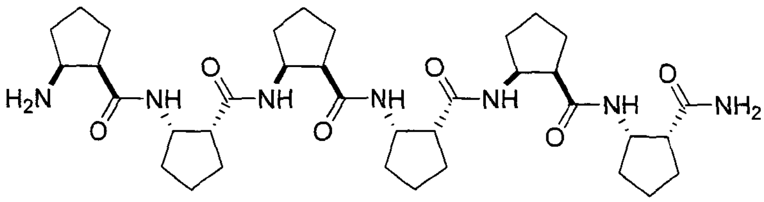

The synthesis of the peptide was performed in two different ways. In the first case the synthesis was performed on Tentagel RRAM resin (Rapp Polymere) with method of the present invention in a continuous flow reactor with 1.5 amino acids equivalents at 60 bar pressure and at 70 °C temperature (flow rate 0.1 ml/min). In the second case the synthesis was performed on the same resin, on the same temperature, with the same coupling agent, with the same amino acids, with the same amino acid equivalents in a microwave reactor. The total yield of the 6 couplings with the method of the present invention is 87%.

**Table 8.** Synthesis of *cis*-2-aminocyclopentanecarboxylic acid hexamer with monotonous chirality

| Example | Method | Temperature (°C) | Amino acid equivalents | Coupling efficiency (C%) |
|---|---|---|---|---|
| 39 | Method of the invention (60 bar pressure) | 70 | 1.5 | 87 |
| 40 (comparative) | Microwave (without pressure) | 70 | 1.5 | 32 |

**Examples 41-42**

Synthesis of synaptotoxic β-amyloid neutralizing β-peptide conjugate fragment ([1S,2S]-ACHC-β$^3$-*h*Arg-([1S,2S]-ACHC)$_2$-β$^3$-*h*Asp-[1S,2S]-ACHC-Gly-Gly-Cys-NH$_2$)

The 2-aminocyclohexanecarboxylic acid (ACHC) containing β-peptide conjugate neutralizes the synaptotoxic β-amyloid (Fulop, L.; Mandity, I. M.; Juhasz, G.; Szegedi, V.; Hetenyi, A.; Weber, E.; Bozso, Z.; Simon, D.; Benko, R.; Kiraly, Z.; Martinek, T. A. Plos One 2012, 7), the structure of the key element of the synthesis is shown below.

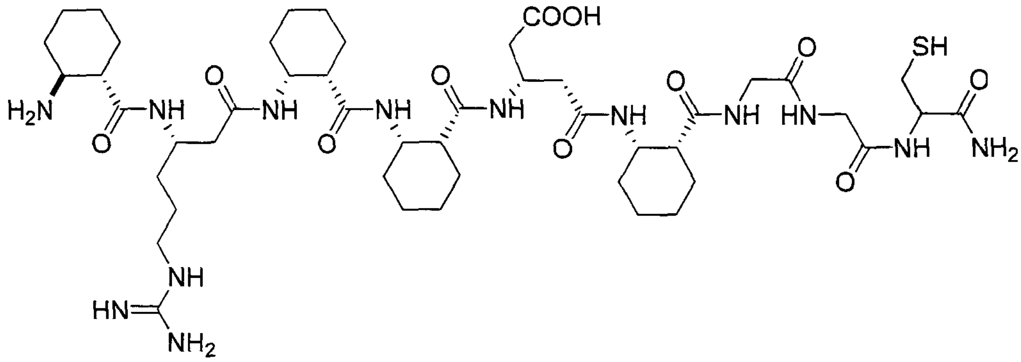

The coupling of ACHC is especially difficult and can be completed only with double coupling (Justin K. Murray, Bilal Farooqi, Jack D. Sadowsky, Mark Scalf, Wesley A. Freund, Lloyd M. Smith, Jiandong Chen, Samuel H. Gellman J. AM. CHEM. SOC. 2005, 127, 13271- -13280).

The synthesis of the peptide was performed in two different ways. In the first case the synthesis was performed on Tentagel RRAM resin (Rapp Polymere) with method of the present invention in a continuous flow reactor with 1.5 amino acids equivalents at 60 bar pressure and at 70 °C temperature (flow rate 0.1 ml/min). In the second case the synthesis was performed on the same resin, on the same temperature, with the same coupling agent, with the same amino acids, with the same amino acid equivalents in a microwave reactor. The total yield of the 9 couplings with the method of the present invention is 97%.

**Table 9.** Synthesis of synaptotoxic β-amyloid neutralizing β-peptide conjugate fragment

| Example | Method | Temperature (°C) | Amino acid equivalents | Coupling efficiency (C%) |
|---|---|---|---|---|
| 41 | Method of the invention (60 bar pressure) | 70 | 1.5 | 97 |
| 42 (comparative) | Microwave (without pressure) | 70 | 1.5 | 28 |

The biologically active peptides obtained by the method of the present invention can be transformed into medicine with usual methods of formulation.

**Examples 43-44**

Synthesis of cell penetrating gamma peptide H-(GAAP)6-$NH_2$

The gamma-aminoproline derivatives possess cell penetrating effect: Farrera-Sinfreu J, Giralt E, Castel S, Albericio F, Royo M **2005,** *127*, 9459-9468.

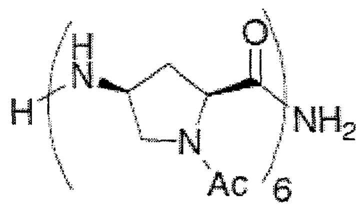

The synthesis of the peptide was performed in two different ways. In the first case the synthesis was performed on Tentagel RRAM resin (Rapp Polymere) with method of the present invention in a continuous flow reactor with 1.5 amino acids equivalents on 60 bar pressure on 70 °C temperature (flow rate 0.1 ml/min). In the second case the synthesis was performed on the same resin, on the same temperature, with the same coupling agent, with the same amino acids, with the same amino acid equivalents in a microwave reactor. The total yield of the 6 couplings with the method of the present invention is 92%.

**Table 10.** Synthesis of cell penetrating gamma peptide

| Example | Method | Temperature (°C) | Amino acid equivalents | Coupling efficiency (C %) |
|---|---|---|---|---|
| 43 | Method of the invention (60 bar pressure) | 70 | 1.5 | 92 |
| 44 (comparative) | Microwave (without pressure) | 70 | 1.5 | 37 |

The biologically active peptide obtained by the method of the present invention can be transformed into medicine with usual methods of formulation.

**Examples 45-46**

Synthesis of capped sermorelin, a fluorescein labelled GH-RH analog Fluoresceinyl-YADAIFTNSYRKVLGQLSARKL-LQDIMSR-$NH_2$ [Prakash A, Goa KL BioDrugs: Clinical Immunotherapeutics, Biopharmaceuticals and Gene Therapy 1999, 12, 139-157].

The cell-membrane penetration of sermorelin can be measured by the application of fluorescein labelling [Beata Kolesinska, Dominika J. Podwysocka, Magnus A. Rueping, Dieter Seebach, Faustin Kamena, Peter Walde, Markus Sauer, Barbara Windschiegl, Mira Meyer-Acs, Marc Vor der Brüggen, and Sebastian Giehring CHEMISTRY & BIODIVERSITY 2013, 10, 1-38].

The synthesis of the peptide was performed in two different ways. In the first case the synthesis was performed on Tentagel RRAM resin (Rapp Polymere) with method of the present invention in a continuous flow reactor with 1.5 amino acids equivalents at 60 bar pressure and at 70 °C temperature (flow rate 0.1 ml/min). In the second case the synthesis was performed on the same resin, on the same temperature, with the same coupling agent, with the same amino acids, with the same amino acid equivalents in a microwave reactor. The total yield of the 29 couplings with the method of the present invention is 78%.

**Table 11.** Synthesis of fluorescein labelled peptide

| Example | Method | Temperature (°C) | Amino acid equivalents | Coupling efficiency (C%) |
|---|---|---|---|---|
| 45 | Method of the invention (60 bar pressure) | 70 | 1.5 | 78 |
| 46 (comparative) | Microwave (without pressure) | 70 | 1.5 | 19 |

The biologically active peptide obtained by the method of the present invention can be transformed into medicine with usual methods of formulation.

**Examples 47-48**

Synthesis of capped γ-secretase inhibiting acetylated ACPC oligomer Ac-([1S,2S]-ACPC)$_{12}$-$NE_2$ [Y. Imamura, N. Umezawa, S. Osawa, N. Shimada, T. Higo, S. Yokoshima, T. Fukuyama, T. Iwatsubo, N. Kato, T. Tomita and T. Higuchi, *Journal of Medicinal Chemistry* **2013,** *56*, 1443-1454.]

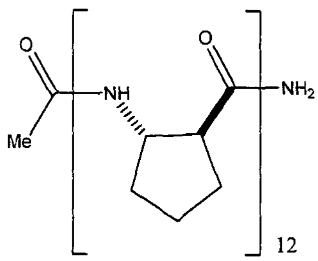

The synthesis of the peptide was performed in two different ways. In the first case the synthesis was performed on Tentagel RRAM resin (Rapp Polymere) with method of the present invention in a continuous flow reactor with 1.5 amino acids equivalents at 60 bar pressure and at 70 °C temperature (flow rate 0.1 ml/min). In the second case the synthesis was performed on the same resin, on the same temperature, with the same coupling agent, with the same amino acids, with the same amino acid equivalents in a microwave reactor. The capping has been performed according to known literature methods [Murray Goodman, Claudio Toniolo, Luis Moroder: Synthesis of Peptides and Peptidomimetics (Methods in Organic Chemistry) Houben-Weyl: Vol. E 22 a-d 2002; Andrew B. Hughes: Amino Acids, Peptides and Proteins in Organic Chemistry, Vol1-5, Wiley-VCH 2011]. The total yield of the 12 couplings and 1 capping step with the method of the present invention is 94%.

**Table 12.** Synthesis of $\gamma$-secretase inhibiting acetylated ACPC oligomer.

| Example | Method | Temperature (°C) | Amino acid equivalents | Coupling efficiency (C %) |
|---|---|---|---|---|
| 47 | Method of the invention (60 bar pressure) | 70 | 1.5 | 94 |
| 48 (comparative) | Microwave (without pressure) | 70 | 1.5 | 26 |

The biologically active peptide obtained by the method of the present invention can be transformed into medicine with usual methods of formulation.

**Examples 49-50**

Synthesis of H-AHPA-Leu-$NH_2$ Bestatine amide [Hirayama, Y; Sakamaki, S; Takayanagi, N; Tsuji, Y; Sagawa, T; Chiba, H; Matsunaga, T; Niitsu, Y *Cancer & chemotherapy* **2003** *30* 1113-1138.]

The synthesis of the peptide was performed in two different ways. In the first case the synthesis was performed on Tentagel RRAM resin (Rapp Polymere) with method of the present invention in a continuous flow reactor with 1.5 amino acids equivalents at 60 bar pressure and at 70 °C temperature (flow rate 0.1 ml/min). In the second case the synthesis was performed on the same resin, on the same temperature, with the same coupling agent, with the same amino acids, with the same amino acid equivalents in a microwave reactor.. The capping has been performed according to known literature methods [Murray Goodman, Claudio Toniolo, Luis Moroder: Synthesis of Peptides and Peptidomimetics (Methods in Organic Chemistry) Houben-Weyl: Vol. E 22 a-d 2002; Andrew B. Hughes: Amino Acids, Peptides and Proteins in Organic Chemistry, Vol1-5, Wiley-VCH 2011].The total yield of the 2 couplings and 1 capping step with the method of the present invention is 97%.

**Table 13.** Synthesis of H-AHPA-Leu-$NH_2$

| Example | Method | Temperature (°C) | Amino acid equivalents | Coupling efficiency (C%) |
|---|---|---|---|---|
| 49 | Method of the invention (60 bar pressure) | 70 | 1.5 | 97 |
| 50 (comparative) | Microwave (without pressure) | 70 | 1.5 | 43 |

**Examples 51-52**

Synthesis of H-AHPA-Leu-OH Bestatine [Hirayama, Y; Sakamaki, S; Takayanagi, N; Tsuji, Y; Sagawa, T; Chiba, H; Matsunaga, T; Niitsu, Y *Cancer & chemotherapy* **2003** *30* 1113-1138.]

The synthesis of the peptide was performed in two different ways. In the first case the synthesis was performed on polystyrene resin with Wang (hydroxymethylphenyl) linker with method of the present invention in a continuous flow reactor with 1.5 amino acids equivalents at 60 bar pressure and at 70 °C temperature (flow rate 0.1 ml/min). In the second case the synthesis was performed on the same resin, on the same temperature, with the same coupling agent, with the same amino acids, with the same amino acid equivalents in a microwave reactor. Performing the cleavage with Wang (hydroxymethylphenyl) linker peptide acid derivative is obtained. The capping has been performed according to known literature methods [Murray Goodman, Claudio Toniolo, Luis Moroder: Synthesis of Peptides and Peptidomimetics (Methods in Organic Chemistry) Houben-Weyl: Vol. E 22 a-d 2002; Andrew B. Hughes: Amino Acids, Peptides and

Proteins in Organic Chemistry, Vol1-5, Wiley-VCH 2011]. The total yield of the 2 couplings and 1 capping step with the method of the present invention is 98%.

**Table 13.** Synthesis of H-AHPA-Leu-OH

| Example | Method | Temperature (°C) | Amino acid equivalents | Coupling efficiency (C %) |
|---|---|---|---|---|
| 51 | Method of the invention (60 bar pressure) | 70 | 1.5 | 98 |
| 52 (comparative) | Microwave (without pressure) | 70 | 1.5 | 57 |

**Claims**

**1.** Continuous flow chemical method for the solid phase synthesis of peptides and enantiomers, diastereomers, stereoisomers, mixtures and salts thereof, and/or derivatives thereof, comprising the steps of:

a) preconditioning the solid support for coupling;
b) coupling an amino acid free on one terminal group and protected on the other terminal group, and optionally on any other reactive group(s), to the solid support; then
c) removing the terminal protecting group from the amino acid coupled to the solid support; then
d) coupling another amino acid free on the corresponding terminal group and protected on the other terminal group, and optionally on any other reactive group(s), to the product obtained in step c); then
repetition of the operations of steps c) and d) in succession on the protected terminal amino acid of the product obtained in each step d) as many times as necessary to obtain the desired product; and then optionally
e) after removing the terminal protecting group from the product obtained in the final step d) "capping" the product thus obtained with a compound free or activated on a reactive group and protected on any other reactive group(s); and/or
f) cleaving the peptide obtained in steps d) or e) from the solid support, and optionally converting the product obtained in step f) into a salt and/or a derivative, and/or hydrolysis thereof,

**characterized by that** the steps a)-e) are performed on a temperature ranging from 30 °C to 150 °C and on a pressure ranging from 20 bar to 500 bar,and in step d) the molar ratio between the product obtained in step c) and the amino acid to be coupled is not more than 1:1,5.

**2.** The method according to claim 1, **characterized by that** the step a) comprises the operations:

a1) deprotection of the linker attached to the solid support and washing the solid support containing the free linker; or
a2) neutralization of the functional group of the linker attached to the solid support and washing the solid support; or
a3) washing the solid support containing the free linker.

**3.** The method of claim 1 or claim 2 **characterized by that** steps a)-e) are performed at 70 °C temperature and at 60 bar pressure.

**4.** The method according to any of claims 1 to 3 **characterized by that** the ratio of the product obtained in step c) and of the amino acid to be coupled in step d) is at least 1:1.

**5.** The method according to any of claims 1 to 4 **characterized by that** the method is performed with 2,4-dimetoxybenzhydrylamine linker on polystyrene-polyethylene glycol copolymer or polyethylene glycol resin as solid support.

**6.** The method according to any of claims 1 to 5 **characterized by that** in steps b), d) and e) the coupling agent is selected from the group of compounds consisting of carbodiimides, preferentially dicyclohexyl-carbodiimide, diisopropyl-carbodiimide, carbonyl-diimidazol, N-carboxy anhydrides, active ester forming agents, preferentially hydroxysuccinimide, hydroxybenzotriazole, N-hydroxy-5-norbornene-2,3-dicarboxymide, 1-hydroxy-7-azabenzotriazole, pentafluorophenol, amino acid fluoride forming agents, preferentially tetramethyl-fluoroformamidinium hexafluorophosphate, amino acid chloride forming agents, preferentially tetramethyl-chloroformamidinium hexafluorophosphate, phosphonium reagents, preferentially benzotriazole-1-yloxytris(dimethylamino)phosphonium hexafluorophosphate, benzo-triazole-1-yloxytripyrrolidinophosphonium hexafluorophosphate, azabenzotriazole-1-yloxytripyrrolidinophosphonium hexafluorophosphate, bromo-tripyrrolidino-phosphonium hexafluorophosphate, 3-(diethoxyphosphoryloxy)-1,2,3-benzotriazine-4(3H)-one, uronium reagents, preferentially N,N,N',N'-tetramsthyl-O-(1H-benzotriazole-1-yl)uronium hexafluorophosphate, (1-[bis(dimethylamino)-methylene]-1H-1,2,3-triazolo[4,5-*b*]pyridinium 3-oxyde hexafluorophosphate), N,N,N',N'-te!tramethyl-O-(1H-benzotriazole-1-yl)uronium-tetrafluorborate, 1-[(di-methylamino)-(dimethyliminium)-methyl]-1H-1,2,3-triazolo[4,5-b]pyridine-3-oxyde-hexafluorophosphate, O-(azabenzotriazole-1-yl)-1,1,3,3-tetramethyleneuronium-hexafluorophosphate, O-(azabenzotriazole-1-yl)-1,1,3,3-pentamethyleneuronium-hexafluorophosphate, O-(7-azabenzotriazole-1-yl)-1,3-dimethyl-1,3-dimethyleneuronium, and oxyma reagents, preferentially ethyl-(hydroxyimino)cianoacetate, (1-ciano-2-etoxy-2-oxoethylideneaminooxy)dimethylamino-morfolino-carbenium hexafluorophosphate, O-[(etoxycarbonyl)cianomethyleneamino]-NN,N',N'-tetramethyl-uronium-hexafluorophosphate; more preferentially the coupling agent is selected from 1-[bis(dimethylamino)methylene]-1H-1,2,3-triazolo[4,5-*b*]pyridinium-3-oxyde-hexafluorophosphate and ethyl-(hydroxyimino)-cianoacetate.

7. The method according to any of claims 1 to 6 **characterized by that** the method is performed in organic solvents such as hydrocarbons, halogenated hydrocarbons, ketones, alcohols, ethers, nitriles, amides or amine type solvents, and in admixtures or mixtures thereof, preferentially in hexane, diethyl ether, dioxane, tetrahydrofurane, N,N-dimethyl-formamide, N-methyl-pyrrolidon, acetonitrile or N,N-diisopropylethylamine.

8. The method according to any of claims 1 to 7 **characterized by that** the amino acid coupled in step b) and the amino acid(s) coupled in step(s) d) are selected in any number and any order from the group consisting of A, C, D, E, F, G, H, I, K, L, M, N, P, Q, R, S, T, V, W, Y, ACPC, ACHC, β-hAsp, β-hArg, D-naphthylalaninee, D-4-chlorophenylalaninee, D-(3'-pyridyl)-alanine-D-citrulline-D-alanine, D-Leu, Pyroglutamine, S-iso-propylaminoglycine, D-4-carbamido-phenylalaninee, L-(4-(2,6-dioxohexahydropyrimidine-4-carboxamido)-phenylalaninee, D-naphthylalanine, D-Trp, D-Ser(OtBu), D-Arg; Hydroxyproline, thiophenylalanine, (S)-1,2,3,4-tetrahydroisoquinolin-3-carboxylic acid, (3aS,7aS)-octahydro-1H-indole-2-carboxylic acid, (R)-(1-amino-3-methylbuthyl)boronic acid, S-tert-butylglycine, (2*S*,3*R*)-AHPA, (2*S*,3*R*)-α-hydroxy-β-homoleucine, 3-amino-2-hydroxydecanoic acid, NMeTyr, desaminocystein, D-Tyr(Et), homoarginine, (2*S*,3*R*,4*R*,6*E*)-3-hydroxy-4-methyl-2-(methylamino)oct-6-ene carboxylic acid, S-ethylglycine, *N*-methylglycine, *N*-methyl-L-leucine, D-alanine, *N*-methyl-L-valine, NMeGly, L-ornithine, 3-anthranyloyl-L-alanine, β-hydroxyglutamic acid, aminoglycine, (2S,3S)-Fmoc-AHPBA, and Fmoc-GAAP, where the amino acids optionally are protected on the N- or C-terminal groups and optionally on reactive group(s) in the side chain(s).

9. The method according to any of claims 1 to 8 **characterized by that** the operations of steps c) and d) on the protected terminal amino acid of the product obtained in each step d) are repeated at least one time in succession.

10. The method according to claim 9 **characterized by that** the operations of steps c) and d) on the protected terminal amino acid of the product obtained in each step d) are repeated by 1-100 times in succession.

11. The method according to any of claims 1 to 10 **characterized by that** the step e) is performed with hydroxysuccinimide esters or in the case of free carboxylic acid functional group with known coupling agents, or with carboxylic acid derivatives, preferentially with carboxylic acid chlorides, carboxylic acid anhydrides resulting in the formation of acylated derivatives.

12. The method according to claim 11 **characterized by that** step e) is performed with carboxyfluorescein or acetic anhydride in the presence of a coupling agent resulting in the formation of a fluorescein labelled or acetylated derivative.

13. The method according to any of claims 1 to 12 **characterized by that** for the synthesis of peptide acid derivatives the cleavage in step f) is performed with hydrogen fluoride or with trifluoromethanesulfonic acid in case of chloromethylphenyl, phenyl-acetamidomethyl linker; or with trifluoroacetic acid in case of hydroxymethylphenyl, methoxy-hydroxymethylphenyl, chloro-triphenylmethyl-chloride linker; or for the synthesis of peptide amide derivatives with hydrogen fluoride or with trifluoromethanesulfonic acid in case of methylbenzhydrylamine linker; or with trifluoroacetic acid in case of 2,4-dimethoxybenzhydrylamine linker; or with methanolic ammonia solution or with primary and secondary amines in case of hydroxymethylbenzamide linker; or for the synthesis of peptide hydrazide derivatives with hydrazines, or for the synthesis of peptide ester derivatives with alcohols, or for the synthesis of peptide alcohol derivatives with lithium borohydride in the case of hydroxymethylbenzamide linker.

**14.** The method according to claim 13 **characterized by that** for the synthesis peptide acid derivatives in case of hydroxymethylphenyl linker and for the synthesis peptide amide derivatives in case of 2,4-dimethoxybenzhydrylamine linker the cleavage in step f) is performed with trifluoroacetic acid,

**15.** The method according to any of claims 1 to 14 for the synthesis of ALFE-$NH_2$, RLFE-$NH_2$, DLFE-$NH_2$, NLFE-$NH_2$, YLFE-$NH_2$, TLFE-$NH_2$, FLFE-$NH_2$, PLFE-$NH_2$, QLFE-$NH_2$, MLFE-$NH_2$, ELFE-$NH_2$, KLFE-$NH_2$, VLFE-$NH_2$, GLFE-$NH_2$, CLFE-$NH_2$, ILFE-$NH_2$, LLFE-$NH_2$. SLFE-$NH_2$, WLFE-$NH_2$, HLFE-$NH_2$, VQAAIDYING-$NH_2$, GILTVSVAV-$NH_2$, H-([1S,2R]-ACPC-[1R,2S]-ACPC)$_3$-$NH_2$, H-([1R,2S]-ACPC)$_6$-$NH_2$, [1S,2S]-ACHC$\beta^3$-*h*Arg-([1S)2S]-ACHC)$_2$-$\beta^3$-*h*Asp-[1S)2S]-ACHC-Gly-Gly-Cys-$NH_2$, H-(GAAP)$_6$-$NH_2$, Fluoresceinyl-YADAIFTNSYRKVLGQLSARKL-LQDIMSR-$NH_2$, Ac-([1S,2S]-ACPC)$_{12}$-$NH_2$, H-AHPA-Leu-$NH_2$ peptides **characterized by that** the appropriate starting materials are used.

**16.** The method according to any of claims 1 to 14 for the synthesis of a peptide selected from the group consisting of
Arg$_{5-8}$,
RQIKIWFQNRRMKWKK,
ACPC-ACPC,
ACHC-ACHC,
ACTH(1-24),
SYSMEHFRWGKPVGKKRRPVKVYP,

H-His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr-OH,

H--His-Ser-Asp-Gly-Thr-Phe-Thr-Ser-Glu-Leu-Ser-Arg-Leu-Arg-Asp-Ser-Ala-Arg-Leu-Gln-Arg-Leu-Leu-Gln-Gly-Leu-Val–$NH_2$,

piroGlu-His-Trp-Ser-Tyr-Gly-Leu-Arg-Pro-Gly-$NH_2$,
acetyl-D-3-(2'-naphthyl)-alanine-D-4-chlorophcnylalanine-D-3-(3'-pyridyl)-alanine-L-sterine-L-tyrozine-D-citrulline-L-leucine-L-arginine-L-proline-D-alanine-amide,
Pyr-His-Trp-Ser-Tyr-D-Leu-Leu-Arg-Pro-NHEt, (4R)-N-{4-[(2S)-2-{[(1R)-2-[4-(carbamoylamino)phenyl]-1-{[(1S)-1-{[(2S)-1-(2-{[(1R)-1-carbamoylethyl]carbamoyl)pyrrolidin-1-yl)-1-oxo-6-[(propane-2-yl)atnino]hexane-2-yl] carbamoyl}-3-methylbutyl]carbamoyl}ethyl]carbamoyl}-2-[(2S)-2-[(2R)-2-[(2R)-3-(4-chlorophenyl)-2-[(2R)-2-acetamido-3-(naphthalene-2-yl)propaneamido]propaneamido]-3-(pyridin-3-yl)propane-amido]-3-hyroxypropaneamido]ethyl}-2,6-dioxo-1,3-diazinane-4-carboxamide,
(2S)-N-[(2S)-1-[[(2S)-1-[[(2S)-1-[[(2S)-1-[[(2R)-1-[[(2S)-1-[[(2S)-5-(diaminomethylidene-amino)-1-[(2S)-2-(ethylcarbamoyl)pyrrolidin-1-yl]-1-oxopentane-2-yl]amino]-4-methyl-1-oxopentan-2-yl]amino]-3-(*1H*-indol-3-yl)-1-oxopropane-2-yl]amino]-3-(4-hyroxyphenyl)-1-oxopropane-2-yl]amino]-3-hyroxy-1-oxopropane-2-yl]amino]-3-(1H-indol-3-yl)-1-oxopropane-2-yl]amino]-3-(*1H*-imidazol-5-yl)-1-oxo-propane-2-yl]-5-oxopirrolidin-2-carboxamide,
Pyr-His-Trp-Ser-Tyr-D-Trp-Leu-Arg-Pro-Gly-$NH_2$,
Pyr-His-Trp-Ser-Tyr-D-Ser(OtBu)-Leu-Arg-Pro-NH-NH-CO-$NH_2$,
Pyr-His-Trp-Ser-Tyr-D-Ser(OtBu)-Leu-Arg-Pro-NHEt,

H-Ser-Val-Ser-Glu-yle-Gln-Leu-Met-His-Asn-Leu-Gly-Lys-His-Leu-Asn-Ser-Met-Glu-Arg-Val-Glu-Trp-Leu-Arg-Lys-Lys-Leu-Gln-Asp-Val-His-Asn-Phe-OH,

SQEPPISLDLTFHLLREVLEMTKADQLAQQAHSNRKLLDIA,

HO-Tyr-Ala-Asp-Ala-ylc-Phe-Thr-Asn-Ser-Tyr-Arg-Lys-Val-Leu-Gly-Gln-Leu-Ser-Ala-Arg-Lys-Leu-Leu-Gln-Asp-yle-Met-Ser-Arg-Gln-Gln-Gly-Glu-Ser-Asn-Gln-Glu-Arg-Gly-Ala-Arg-Ala-Arg-Leu-$NH_2$,

HO-Gln-Leu-Ser-Ala-Arg-Lys-Leu-Leu-Gln-Asp-yle-Met-Ser-Arg-Gln-Gln-Gly-Glu-Ser-Asn-Gln-Glu-Arg-Gly-Ala-Arg-Ala-Arg-Leu-$NH_2$,

Pyr-His-Pro-$NH_2$,
SDAAVDTSSEITTKDLKEKKEVVEEAEN,
RLDVY,
HGEGTFTSDLSKQMEEEAVRLFIEWLKNGGPSSGAPPPS,

H-His-Ala-Glu-Gly-Thr-Phe-Thr-Ser-Asp-Val-Ser-Ser-Tyr-Leu-Glu-Gly-Gln-Ala-Ala-Lys(γ-Glu-palmitoyl)-Glu-Phe-yle-Ala-Trp-Leu-Val-Arg-Gly-Arg-Gly-OH,

FPRPGGGGNGDFEEIPEEIL,
Pyr-His-Trp-Ser-Tyr-Gly-Leu-Arg-Pro-Gly-$NH_2$,
D-Arg-Arg-ProHyp-Gly-Thi-Ser-D-Tic-Oic-Arg,
YADAIFTNSYRKVLGQLSARKLLQDIMSRQ,
[(1R)-3-methyl-1-({(2S)-3-phenyl-2-[(pyrazin-2-ylcarbonyl)amino]propanoyl}amino)butyl]-boronic acid,
N-[2,2-dimethyl-1-(methylcarbamoyl)-propyl]-2-[hydroxy-(hydroxycarbamoyl)methyl]-4-methyl-pentaneamide,
GIGKFLKKAKKFGKAFVKILKK,
GIGKFLHSAKKFGKAFVGEIMNS,
IB367,
RGGLCYCRGRFCVCVGR,
LRWPWWWPWRRK-$NH_2$,
(2*S*,3*R*)-AHPA-L-Leu,
(2*S*,3*R*)-AHPA-L-Pro-L-Pro-L-Ala-$NH_2$, (2*S*,3*R*)-3-amino-2-hyroxy-5-methylhexanoyl-L-Val-L-val-L-Asp-OH,
(2*S*,3*R*)-AHPA-Val-Phe-OH,
(2*S*,3*R*)-Leu-Pro-Pro-OH,
3-amino-2-hyroxy-decanoyl-Ala-Val-NMeTyr-Tyr-OH.
CYIQNCPLG-$NH_2$,
3-Mercaptopropionyl-D-Tyr(Et)-yle-Thr-Asn-Cys-Pro-Orn-Gly-$NH_2$,
H-Cys-Tyr-Phe-Gln-Asn-Cys-Pro-Arg-Gly-$NH_2$, *desamino*-Cys-Tyr-Phe-Gln-Asn-Cys-Pro-D-Arg-Gly-$NH_2$,
$N^6$-(aminoiminometyl)-$N^2$-(3-mercapto-1-oxopropyl)-L-lysyl-glycyl-L-α-aspartyl-L-tryptophyl-L-prolyl-L-cisteinamide, KCNTATCATQRLANFLVHSSNNFGPILPPTNVGSNTY-$NH_2$, H-[(2*S*,3*R*,4*R*,6*E*)-3-hyroxy-4-methyl-2-(methylamino)oct-6-enoyl]-L-2-aminobutanoyl-*N*-methylglycyl-*N*-methyl-L-leucyl-L-valyl-*N*-methyl-L-leucy-L-alanyl-D-alanyl-*N*-methyl-L-leucyl-N-methyl-L-leucyl-*N*-methyl-L-valine-OH,
Ac-YTSLIHSLIEESQNQQEKNEQELLELDKWASLWNWF-$NH_2$,
H-Val-Pro-NMeGly-OH,
H-D-Phe-Cys-Phe-D-Trp-Lys-Thr-Cys-Thr(ol),
N-decanoyl-L-triptophyl-L-asparaginyl-L-aspartyl-L-treonylglicyl-L-omityl-L-aspartyl-D-alanyl-L-aspartylglicyl-D-seryl-threo-3-methyL-L-glutamyl-3-anthranyloyl-L-alanine, H-Val-Orn-Leu-D-Phe-Pro-Val-Orn-Leu-D-Phe-Pro-OH
**characterized by that** the appropriate starting materials are used.

## Patentansprüche

1. Chemisches Durchflussverfahren zur Festphasensynthese von Peptiden und Enantiomeren, Diastereomeren, Stereoisomeren, Gemischen und Salzen davon, und/oder Derivaten davon, umfassend die Schritte:

   a) Vorkonditionieren des festen Trägers für das Kuppeln;
   b) Kuppeln einer Aminosäure, die an einer terminalen Gruppe frei und an der anderen Endgruppe und gegebenenfalls an jeglicher/jeglichen anderen reaktiven Gruppe(n) geschützt ist, an den festen Träger; dann
   c) Entfernen der terminalen Schutzgruppe von der an den festen Träger gekuppelten Aminosäure; dann
   d) Kuppeln einer anderen Aminosäure, die an der entsprechenden terminalen Gruppe frei und an der anderen terminalen Gruppe und gegebenenfalls an jeglicher/jeglichen anderen reaktiven Gruppe(n) geschützt ist, an das in Schritt c) erhaltene Produkt; dann

Wiederholung der Arbeitsgänge der Schritte c) und d) nacheinander an der geschützten terminalen Aminosäure des in jedem Schritt d) erhaltenen Produkts so oft wie nötig, um das gewünschte Produkt zu erhalten; und dann gegebenenfalls
e) nach Entfernen der terminalen Schutzgruppe von dem im letzten Schritt d) erhaltenen Produkt "Abschließen" des so erhaltenen Produkts mit einer Verbindung, die an einer reaktiven Gruppe frei oder aktiviert ist und an jeglicher/jeglichen anderen reaktiven Gruppe(n) geschützt ist; und/oder
f) Abspalten des in den Schritten d) oder e) erhaltenen Peptids von dem festen Träger und gegebenenfalls Umwandeln des in Schritt f) erhaltenen Produkts in ein Salz und/oder ein Derivat, und/oder Hydrolyse davon,

**dadurch gekennzeichnet, dass** die Schritte a)-e) bei einer Temperatur im Bereich von 30 °C bis 150 °C und bei einem Druck im Bereich von 20 bar bis 500 bar durchgeführt wird und in Schritt d) das Molverhältnis zwischen dem in Schritt c) erhaltenen Produkt und der zu kuppelnden Aminosäure nicht mehr als 1:1,5 beträgt.

2. Verfahren nach Anspruch 1, **dadurch gekennzeichnet, dass** der Schritt a) die folgenden Arbeitsgänge umfasst:

a1) Entschützen des an dem festen Träger gebundenen Linkers und Waschen des den freien Linker enthaltenden festen Trägers; oder
a2) Neutralisieren der funktionellen Gruppe des an den festen Träger gebundenen Linkers und Waschen des festen Trägers; oder
a3) Waschen des festen Trägers, der den freien Linker enthält.

3. Verfahren nach Anspruch 1 oder Anspruch 2, **dadurch gekennzeichnet, dass** die Schritte a)-e) bei einer Temperatur von 70 °C und einem Druck von 60 bar durchgeführt werden.

4. Verfahren nach einem der Ansprüche 1 bis 3, **dadurch gekennzeichnet, dass** das Verhältnis des in Schritt c) erhaltenen Produkts und der in Schritt d) zu kuppelnden Aminosäure mindestens 1:1 beträgt.

5. Verfahren nach einem der Ansprüche 1 bis 4, **dadurch gekennzeichnet, dass** das Verfahren mit einem 2,4-Dimethoxybenzhydrylamin-Linker auf Polystyrol-Polyethylenglykol-Copolymer oder Polyethylenglykolharz als festem Träger durchgeführt wird.

6. Verfahren nach einem der Ansprüche 1 bis 5, **dadurch gekennzeichnet, dass** in den Schritten b), d) und e) das Kupplungsmittel aus der Gruppe von Verbindungen ausgewählt ist, die aus Carbodiimiden, vorzugsweise Dicyclohexylcarbodiimid, Diisopropylcarbodiimid, Carbonyldiimidazol, N-Carboxyanhydriden, aktiven Esterbildnern, vorzugsweise Hydroxysuccinimid, Hydroxybenzotriazol, N-Hydroxy-5-norbornen-2,3-dicarbonsäureamid, 1-Hydroxy-7-azabenzotriazol, Pentafluorphenol, Aminosäurefluorid bildenden Mitteln, vorzugsweise Tetramethylfluorformamidiniumhexafluorophosphat, Aminosäurechlorid bildenden Mitteln, vorzugsweise Tetramethylchlorformamidiniumhexafluorophosphat, Phosphoniumreagenzien, vorzugsweise Benzotriazol-1-yloxytris(dimethylamino)phosphoniumhexafluorophosphat, Benzotriazol-1-yloxytripyrrolidinophosphoniumhexafluorphosphat, Azabenzotriazol-1-yloxytripyrrolidinophosphoniumhexafluorphosphat, Bromtripyrrolidinophosphoniumhexafluorophosphat, 3-(Diethoxyphosphoryloxy)-1,2,3-benzotriazin-4(3H)-on, Uroniumreagenzien, vorzugsweise N,N,N',N'-Tetramethyl-O-(1H-benzotriazol-1-yl)uroniumhexafluorophosphat, (1-[Bis(dimethylamino)methylen]-1H-1,2,3-triazolo[4,5-*b*]pyridinium-3-oxid-Hexafluorophosphat), N,N,N',N'-Tetramethyl-O-(1H-benzotriazol-1-yl)uroniumtetrafluorborat, 1-[(Dimethylamino)-(dimethyliminium)methyl]-1H-1,2,3-triazolo[4,5-*b*]pyridin-3-oxid-Hexafluorophosphat, O-(Azabenzotriazol-1-yl)-1,1,3,3-tetramethylenuroniumhexafluorophosphat, O-(Azabenzotriazol-1-yl)-1,1,3,3-pentamethylenuroniumhexafluorophosphat, O-(7-Azabenzotriazol-1-yl)-1,3-dimethyl-1,3-dimethylenuronium und Oxymareagenzien, vorzugsweise Ethyl(hydroxyimino)cyanoacetat, (1-Cyano-2-ethoxy-2-oxoethylidenaminooxy)dimethylaminomorpholinocarbeniumhexafluorophosphat, O-[(Ethoxycarbonyl)cyanomethylenamino]-N,N,N',N'-tetramethyluroniumhexafluorophosphat besteht;
stärker bevorzugt ist das Kupplungsmittel ausgewählt aus 1-[Bis(dimethylamino)methylen]-1H-1,2,3-triazolo[4,5-*b*]pyridinium-3-oxid-Hexafluorphosphat und Ethyl(hydroxyimino)cyanoacetat.

7. Verfahren nach einem der Ansprüche 1 bis 6, **dadurch gekennzeichnet, dass** das Verfahren in organischen Lösungsmitteln wie Kohlenwasserstoffen, halogenierten Kohlenwasserstoffen, Ketonen, Alkoholen, Ethern, Nitrilen, Amiden oder Lösungsmitteln vom Amintyp und bevorzugt in Beimischungen oder Gemischen davon durchgeführt wird, vorzugsweise in Hexan, Diethylether, Dioxan, Tetrahydrofuran, N,N-Dimethylformamid, N-Methylpyrrolidon, Acetonitril oder N,N-Diisopropylethylamin.

**8.** Verfahren nach einem der Ansprüche 1 bis 7, **dadurch gekennzeichnet, dass** die in Schritt b) gekuppelte Aminosäure und die in Schritt/in den Schritten d) gekuppelte(n) Aminosäure(n) in beliebiger Anzahl und Reihenfolge aus der Gruppe ausgewählt sind, die aus A, C, D, E, F, G, H, I, K, L, M, N, P, Q, R, S, T, V, W, Y, ACPC, ACHC, β-hasp, β-hArg, D-Naphthylalanin, D-4-Chlorphenylalanin, D-(3'-Pyridyl)alanin-D-citrullin-D-alanin, D-Leu, Pyroglutamin, S-Isopropylaminoglycin, D-4-Carbamidophenylalanin, L-(4-(2,6-Dioxohexahydropyrimidin-4-carboxamido)phenylalanin, D-Naphthylalanin, D-Trp, D-Ser(OtBu), D-Arg; Hydroxyprolin, Thiophenylalanin, (S)-1,2,3,4-Tetrahydroisochinolin-3-carbonsäure, (3aS,7aS)-Octahydro-1H-indol-2-carbonsäure, (R)-(1-Amino-3-methylbutyl)boronsäure, S-tert-Butylglycin, (2*S*,3*R*)-AHPA, (2S,3R)-a-Hydroxy-β-homoleucin, 3-Amino-2-hydroxydecansäure, NMeTyr, Desaminocystein, D-Tyr(Et), Homoarginin, (2S,3R,4R,6E)-3-Hydroxy-4-methyl-2-(methylamino)oct-6-encarbonsäure, S-Ethylglycin, *N*-Methylglycin, *N*-Methyl-L-leucin, D-Alanin, *N*-Methyl-L-valin, NMeGly, L-Ornithin, 3-Anthranyloyl-L-alanin, β-Hydroxyglutaminsäure, Aminoglycin, (2S,3S)-Fmoc-AHPBA und Fmoc-GAAP besteht, wobei die Aminosäuren gegebenenfalls an den N- oder C-terminalen Gruppen und gegebenenfalls an (einer) reaktiven Gruppe(n) in der (den) Seitenkette(n) geschützt sind.

**9.** Verfahren nach einem der Ansprüche 1 bis 8, **dadurch gekennzeichnet, dass** die Arbeitsgänge der Schritte c) und d) an der geschützten terminalen Aminosäure des in jedem Schritt d) erhaltenen Produkts mindestens einmal nacheinander wiederholt werden.

**10.** Verfahren nach Anspruch 9, **dadurch gekennzeichnet, dass** die Arbeitsgänge der Schritte c) und d) an der geschützten terminalen Aminosäure des in jedem Schritt d) erhaltenen Produkts 1-100 Mal nacheinander wiederholt werden.

**11.** Verfahren nach einem der Ansprüche 1 bis 10, **dadurch gekennzeichnet, dass** der Schritt e) mit Hydroxysuccinimidestern oder im Fall einer freien funktionellen Carbonsäuregruppe mit bekannten Kupplungsmitteln oder mit Carbonsäurederivaten, vorzugsweise mit Carbonsäurechloriden, Carbonsäureanhydriden durchgeführt wird, die zur Bildung von acylierten Derivaten führen.

**12.** Verfahren nach Anspruch 11, **dadurch gekennzeichnet, dass** Schritt e) mit Carboxyfluorescein oder Essigsäureanhydrid in Gegenwart eines Kupplungsmittels durchgeführt wird, was zur Bildung eines fluoresceinmarkierten oder acetylierten Derivats führt.

**13.** Verfahren nach einem der Ansprüche 1 bis 12, **dadurch gekennzeichnet, dass** zur Synthese von Peptidsäurederivaten die Spaltung in Schritt f) mit Fluorwasserstoff oder im Fall eines Chlormethylphenyl-, Phenylacetamidomethyl-Linkers mit Trifluormethansulfonsäure durchgeführt wird; oder

im Fall eines Hydroxymethylphenyl-, Methoxyhydroxymethylphenyl-, Chlortriphenylmethylchlorid-Linkers mit Trifluoressigsäure; oder
für die Synthese von Peptidamidderivaten mit Fluorwasserstoff oder im Fall eines Methylbenzhydrylamin-Linkers mit Trifluormethansulfonsäure; oder
im Fall eines 2,4-Dimethoxybenzhydrylamin-Linkers mit Trifluoressigsäure; oder
im Falle eines Hydroxymethyibenzamid-Linkers mit methanolischer Ammoniaklösung oder mit primären und sekundären Aminen; oder
für die Synthese von Peptidhydrazidderivaten mit Hydrazinen oder für die Synthese von Peptidesterderivaten mit Alkoholen oder für die Synthese von Peptidalkoholderivaten mit Lithiumborhydrid im Fall von Hydroxymethylbenzamid-Linkern durchgeführt wird.

**14.** Verfahren nach Anspruch 13, **dadurch gekennzeichnet, dass** für die Synthese Peptidsäurederivate im Fall eines Hydroxymethylphenyl-Linkers und für die Synthese Peptidamidderivate im Fall eines 2,4-Dimethoxybenzhydrylamin-Linkers die Spaltung in Schritt f) mit Trifluoressigsäure durchgeführt wird.

**15.** Verfahren nach einem der Ansprüche 1 bis 14 zur Synthese von ALFE-$NH_2$, RLFE-$NH_2$, DLFE-$NH_2$, NLFE-$NH_2$, YLFE-$NH_2$, TLFE-$NH_2$, FLFE-$NH_2$, PLFE-$NH_2$, QLFE-$NH_2$, MLFE-$NH_2$, ELFE-$NH_2$, KLFE-$NH_2$, VLFE-$NH_2$, GLFE-$NH_2$, CLFE-$NH_2$, ILFE-$NH_2$, LLFE-$NH_2$, SLFE-$NH_2$, WLFE-$NH_2$, HLFE-$NH_2$, VQAAIDYING-$NH_2$, GILTVSVAV-$NH_2$, H-([1S,2R]-ACPC-[1R,2S]-ACPC)$_3$-$NH_2$, H-([1R,2S]-ACPC)$_6$-$NH_2$, [1S,2S]-ACHC-$\beta^3$-*h*Arg-([1S,2S]-ACHC)$_2$-$\beta^3$-*h*Asp-[1S,2S]-ACHC-Gly-Gly-Cys-$NH_2$, H-(GAAP)$_6$-$NH_2$, Fluoresceinyl-YADAIFTNSYRKVLGQLSARKLLQ-DIMSR-$NH_2$, Ac-([1S,2S]-ACPC)$_{12}$-$NH_2$, H-AHPA-Leu-$NH_2$-Peptiden, die **dadurch gekennzeichnet ist, dass** die geeigneten Ausgangsmaterialien verwendet werden.

**16.** Verfahren nach einem der Ansprüche 1 bis 14 zur Synthese eines Peptids, ausgewählt aus der Gruppe bestehend aus

$Arg_{5-8}$,
RQIKIWFQNRRMKWKK,
ACPC-ACPC,
ACHC-ACHC,
ACTH(1-24),
SYSMEHFRWGKPVGKKRRPVKVYP,

H-His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr-OH,
H-His-Ser-Asp-Gly-Thr-Phe-Thr-Ser-Glu-Leu-Ser-Arg-Leu-Arg-Asp-Ser-Ala-Arg-Leu-Gln-Arg-Leu-Leu-Gln-Gly-Leu-Val-$NH_2$,

piroGlu-His-Trp-Ser-Tyr-Gly-Leu-Arg-Pro-Gly-$NH_2$,
Acetyl-D-3-(2'-naphthyl)alanin-D-4-chlorphenylalanin-D-3-(3'-pyridyl)alanin-L-serin-L-tyrosin-D-citrullin-L-leucin-L-arginin-L-prolin-D-alaminamid,
Pyr-His-Trp-Ser-Tyr-D-Leu-Leu-Arg-Pro-NHEt,
(4R)-N-{4-[(2S)-2-{[(1R)-2-[4-(Carbamoylamino)phenyl]-1-{[(1S)-1-{[(2S)-1-(2-{[(1R)-1-carbamoylethyl]carbarmoyl}pyrrolidin-1-yl)-1-oxo-6-[(propan-2-yl)amino]hexan-2-yl]carbamoyl}-3-methylbutyl]carbamoyl}ethyl]carbamoyl}-2-[(2S)-2-[(2R)-2-[(2R)-3-(4-chlorphenyl)-2-[(2R)-2-acetamido-3-(naphthalin-2-yl)propanamido]propanamido]-3-(pyridin-3-yl)propanamido]-3-hydroxypropanamido]ethyl]phenyl}-2,6-dioxo-1,3-diazinan-4-carboxamid,
(2S)-N-[(2S)-1-[[(2S)-[[(2S)-1-[[(2S)-1-[[(2R)-1-[[(2S)-1-[[(2S)-5-(Diaminomethylidenarnino)-1-[(2S)-2-(ethylcarbamoyl)pyrrolidin-1-yl]-1-oxopentan-2-yl]amino]-4-methyl-1-oxopentan-2-yl]amino]-3-(*1H*-indo)-3-yl)-1-oxopropan-2-yl]amino]-3-(4-hyroxyphenyl)-1-oxopropan-2-yl]amino]-3-hydroxy-1-oxopropan-2-yl]amino]-3-(1H-indol-3-yl)-1-oxopropan-2-yl]amino]-3-(*1H*-oxopropan-2-yl]-5-oxopyrrolidin-2-carboxamid,
Pyr-His-Trp-Ser-Tyr-D-Trp-Leu-Arg-Pro-Gly-$NH_2$,
Pyr-His-Trp-Ser-Tyr-D-Ser(OtBu)-Leu-Arg-Pro-NH-NH-CO-$NH_2$,
Pyr-His-Trp-Ser-Tyr-D-Ser(OtBu)-Leu-Arg-Pro-NHEt,

H-Ser-Val-Ser-Glu-yle-Gln-Leu-Met-His-Agn-Leu-Gly-Lys-His-Leu-Asn-Ser-Met-Glu-Arg-Val-Glu-Trp-Leu-Arg-Lys-Lys-Leu-Gln-Asp-Val-His-Asn-Phe-OH,

SQEPPISLDLTFHLLREVLEMTKADQLAQQAHSNRKLLDIA,

HO-Tyr-Ala-Asp-Ala-yle-Phe-Thr-Asn-Ser-Tyr-Arg-Lys-Val-Leu-Gly-Gln-Leu-Ser-Ala-Arg-Lys-Leu-Leu-Gln-Asp-yle-Met-Ser-Arg-Gln-Gln-Gly-Glu-Ser-Asn-Gln-Glu-Arg-Gly-Ala-Arg-Ala-Arg-Leu-$NH_2$,

HO-Gln-Leu-Ser-Ala-Arg-Lys-Leu-Leu-Gln-Asp-yle-Met-Ser-Arg-Gln-Gln-Gly-Glu-Ser-Asn-Gln-Glu-Arg-Gly-Ala-Arg-Ala-Arg-Leu-$NH_2$,

Pyr-His-Pro-$NH_2$,
SDAAVDTSSEITTKDLKEKKEVVEEAEN,
RLDVY,
HGEGTFTSDLSKQMEEEAVRLFIEWLKNGGPSSGAPPPS,

H-His-Ala-Glu-Gly-Thr-Phe-Thr-Ser-Asp-Val-Ser-Ser-Tyr-Leu-Glu-Gly-Gln-Ala-Ala-Lys(γ-Glu-palmitoyl)-Glu-Phe-yle-Ala-Trp-Leu-Val-Arg-Gly-Arg-Gly-OH,

FPRPGGGGNGDFEEIPEEIL,
Pyr-His-Trp-Ser-Tyr-Gly-Leu-Arg-Pro-Gly-$NH_2$,
D-Arg-Arg-Pro-Hyp-Gly-Thi-Ser-D-Tic-Oic-Arg,
YADAIFTNSYRKVLGQLSARKLLQDIMSRQ,
[(1R)-3-Methyl-1-({(2S)-3-phenyl-2-[(pyrazin-2-ylcarbonyl)amino]propanoyl}amino)butyl]-boronsäüre,
N-[2,2-Dimethyl-1-(methylcarbamoyl)propyl]-2-[hydroxy(hydroxycarbamoyl)methyl]-4-methylpentanamid,
GIGKFLKKAKKFGKAFVKILKK,
GIGKFLHSAKKFGKAFVGEIMNS,
IB367,
RGGLCYCRGRFCVCVGR,
LRWPWWPWRRK-$NH_2$,
(2S,3*R*)-AHPA-L-Leu,
(2S,3*R*)-AHPA-L-Pro-L-Pro-L-Ala-$NH_2$,
(2*S*,3*R*)-3-Amino-2-hyroxy-5-methylhexanoyl-L-Val-L-Val-L-Asp-OH,
(2S,3*R*)-AHPA-Val-Phe-OH,
(2S,3*R*)-Leu-Pro-Pro-OH,
3-Amino-2-hydroxydecanoyl-Ala-Val-NMeTyr-Tyr-OH,
CYIQNCPLG-$NH_2$,
3-Mercaptopropionyl-D-Tyr(Et)-yle-Thr-Asn-Cys-Pro-Orn-Gly-$NH_2$,
H-Cys-Tyr-Phe-Gln-Asn-Cys-Pro-Arg-Gly-$NH_2$,
*Desamino*-Cys-Tyr-Phe-Gln-Asn-Cys-Pro-D-Arg-Gly-$NH_2$,
$N^6$-(Aminoiminomethyl)-$N^2$-(3-mercapto-1-oxopropyl)-L-lysyl-glycyl-L-$\alpha$-aspartyl-L-tryptophyl-L-prolyl-L-cysteinamid,
KCNTATCATQRLANFLVHSSNNFGPILPPTNVGSNTY-$NH_2$,
H-[(2*S*,3*R*,4*R*,6*E*)-8-Hydroxy-4-methyl-2-(methylamino)oct-6-enoyl]-L-2-aminobutanoyl-N-methylglycyl-*N*-methyl-L-leucyl-L-valyl-*N*-methyl-L-leucy-L-alanyl-D-alanyl-*N*-methyl-L-leucyl-*N*-methyl-L-leucyl-*N*-methyl-L-valin-OH,
Ac-YTSLIHSLIEESQNQQEKNEQELLELDKWASLWNWF-$NH_2$,
H-Val-Pro-NMeGly-OH,
HD-Phe-Cys-Phe-D-Trp-Lys-Thr-Cys-Thr(ol),
N-Decanoyl-L-tryptophyl-L-asparaginyl-L-aspartyl-L-threonylglycyl-L-ornityl-L-aspartyl-D-alanyl-L-aspartylglycyl-D-seryl-threo-3-methyl-L-glutamyl-3-anthranyloyl-L-alanin,
H-Val-Orn-Leu-D-Phe-Pro-Val-Orn-Leu-D-Phe-Pro-OH, **dadurch gekennzeichnet, dass** die geeigneten Ausgangsmaterialien verwendet werden.

## Revendications

**1.** Procédé chimique à flux continu pour la synthèse en phase solide de peptides et d'énantiomères, de diastéréoisomères, de-stéréoisomères, de mélanges et de sels de ceux-ci, et/ou de dérivés de ceux-ci, comprenant les étapes consistant à :

a) préconditionner le support solide pour l'accouplement ;
b) coupler un acide aminé libre sur un groupe terminal et protégé sur l'autre groupe terminal, et éventuellement sur tout/tous autre(s) groupe(s) réactif(s), au support solide ; puis
c) éliminer le groupe protecteur terminal de l'acide aminé couplé au support solide ; puis
d) coupler un autre acide aminé libre sur le groupe terminal correspondant et protégé sur l'autre groupe terminal, et éventuellement tout/tous autre(s) groupe(s) réactif(s), au produit obtenu à l'étape c) ; puis
répéter les opérations des étapes c) et d) successivement sur l'acide aminé terminal protégé du produit obtenu à chaque étape d) autant de fois que nécessaire pour obtenir le produit désiré ; et puis éventuellement
e) après avoir éliminé le groupe protecteur terminal du produit obtenu à l'étape finale d), « coiffer » le produit ainsi obtenu avec un composé libre ou activé sur un groupe réactif et protégé sur tout/tous autre(s) groupe(s) réactif(s) ; et/ou
f) cliver le peptide obtenu aux étapes d) ou e) à partir du support solide, et éventuellement convertir le produit obtenu à l'étape f) en un sel et/ou un dérivé, et/ou son hydrolyse, **caractérisé en ce que** les étapes a)-e) sont effectuées à une température allant de 30 °C à 150 °C et à une pression allant de 20 bar à 500 bar, et qu'à l'étape d), le rapport molaire entre le produit obtenu à l'étape c) et l'acide aminé à coupler n'est pas supérieur à 1:1,5.

**2.** Procédé selon la revendication 1, **caractérisé en ce que** l'étape a) comprend les opérations :

a1) de déprotection du lieur fixé au support solide et de lavage du support solide contenant le lieur libre ; ou
a2) de neutralisation du groupe fonctionnel du lieur fixé au support solide et de lavage du support solide ; ou
a3) de lavage du support solide contenant le lieur libre.

**3.** Procédé selon la revendication 1 ou la revendication 2, **caractérisé en ce que** les étapes a) à e) sont effectuées à une température de 70 °C et à une pression de 60 bar.

**4.** Procédé selon l'une quelconque des revendications 1 à 3, **caractérisé en ce que** le rapport entre le produit obtenu à l'étape c) et l'acide aminé à coupler à l'étape d) est d'au moins 1:1.

**5.** Procédé selon l'une quelconque des revendications 1 à 4, **caractérisé en ce que** le procédé est effectué avec un lieur 2,4-dimétoxy-benzhydrylamine sur un copolymère polystyrène-polyéthylèneglycol ou une résine de polyéthylèneglycol comme support solide.

**6.** Procédé selon l'une quelconque des revendications 1 à 5, **caractérisé en ce que,** aux étapes b), d) et e), l'agent de couplage est choisi dans le groupe de composés constitué des carbodiimides, de préférence le dicyclohexylcarbodiimide, le diisopropyl-carbodiimide, le carbonyle-diimidazol, les anhydrides de N-carboxy, des agents de formation d'esters actifs, de préférence l'hydroxysuccinimide, l'hydroxybenzotriazole, le N-hydroxy-5-norbomène-2,3-dicarboxymide, le 1-hydroxy-7-azabenzotriazole, le pentafluorophénol, des agents de formation du fluorure d'acide aminé, de préférence l'hexafluorophosphate de tétraméthylfluoroformamidinium, des agents de formation de chlorure d'acide, de préférence l'hexafluorophosphate de tétraméthyl-chloroformamidinium, des réactifs de phosphonium, de préférence l'hexafluorophosphate de benzo-triazole-1-yloxytris(diméthylamino)phosphonium, l'hexafluorophosphate de benzo-triazol-1-yloxytripyrrolidinophosphonium, l'hexafluorophosphate d'azabenzotriazole-1-yloxytripyrrolidinophosphonium, l'hexafluorophosphate de bromotripyrrolidino-phosphonium, la 3-(diéthoxyphosphoryloxy)-1,2,3-benzotriazine-4(3H)-one, des réactifs d'uronium, de préférence l'hexafluorophosphate de N,N,N',N'-tétraméthyl-O-(1H-benzotriazole-1-yl)uronium, l'hexafluorophosphate de 3-oxyde de (1-[bis(diméthylammo)-méthylene]-1H-1,2,3-triazolo[4,5-b]pyridinium), le N,N,N',N'-tétraméthyl-O-(1H-benzotriazole-1-y1)uronium-tetraffuorborate, le 1-[(di-methylamino)-(dimethylirninium)-methyl]-1H-1,2,3-triazolo[4,5-*b*]pyridine-3-oxyde-hexafluorophosphate, le O-(azabenzotriazole-1-yl)-1,1,3,3-tetraméthylèneuronium-hexafluorophosphate, le O-(azabenzotriazole-1-yl)-1,1,3,3-pentaméthylèneuronium-hexafluorophosphate, le O-(7-azabenzotriazole)-1-yl)-1,3-diméthyl-1,3-diméthylone-uronium, et les réactifs oxyma, de préférence l'éthyl-(hydroxyimino)cianoacétate, l'hexa-fluorophosphate(1-ciano-2-étoxy-2-oxoéthylidènearninooxy)diméthylamino-morfolino-carbénium, l'O-[(éthoxycarbonyl) cianométhylèneamino]-N,N,N',N'-tétraméthyl-uronium-hexafluorophosphate ; et de préférence, l'agent de couplage est choisi parmi le 1-[bis(diméthylamïno)méthylènel-1H-1,2,3-triazolo[4,5-*b*]pyridinium-3-oxyde-hexafluorophosphate et l'éthyl-(hydroxyimino)-cianoacétate.

**7.** Procédé selon l'une quelconque des revendications 1 à 6, **caractérisé en ce que** le procédé est effectué dans des solvants organiques tels que des hydrocarbures, des hydrocarbures halogènes, des cétones, des alcools, des éthers, des nitriles, des amides ou des solvants de type amine, et dans des additifs ou des mélanges de ceux-ci, de préférence dans l'hexane, l'éther diéthylique, le dioxane, le tétrahydrofurane, le N,N-diméthyl-formamide, le N-méthyl-pyrrolidone, l'acétonitrile ou la N,N-diisopropyléthylamine.

**8.** Procédé selon l'une quelconque des revendications 1 à 7, **caractérisé en ce que** l'acide aminé accouplé à l'étape b) et le(s) acide(s) aminé(s) accouplé(s) à l'étape/aux étapes d) sont sélectionnés dans un nombre quelconque et dans un ordre quelconque dans le groupe constitué de A, de C, de D, de E, de F, de G, de H, de I, de K, de L, de M, de N, de P, de Q, de R, de S, de T, de V, de W, de Y, d'ACPC, d'ACHC, de β-hAsp, de β-hArg, de la D-naphtylalanine, de la D-4-chlorophénylalanine, de la D-(3'-pyridyl)-alanine-D-citrulline-D-alanine, de D-Leu, de la pyroglutarnine, de la S-iso-propylaminoglycine, de la D-4-carbamido-phénylalanine, de la L-(4-(2,6-dioxohexahydropyrimidine-4-carboxamido)-phénylalanine, de la D-naphtylalanine, de la D-Trp, de D-Ser (OtBu), de D-Arg ; de l'hydroxyproline, de la thiophénylalanine, de l'acide(S)-1,2,3,4-tétrahydroisoquinoléine-3-carboxylique, de l'acide (3aS, 7aS)-octahydro-1H-indole-2-carboxylique, de l'acide (R)-(1-amino-3-méthylbuthyl)boronique, de la S-tert-butylglycine, de (2S,3R)-AHPA, de la (2S,3R)-a-hydroxy-p-homoleucine, de l'acide 3-amino-2-hydroxydécanoïque, de NMeTyr, de la desamino-cystéine, de D-Tyr(Et), de l'homoarginine, de l'acide carboxylique de (2S,3R,4R,6E)-3-hydroxy-4-méthyl-2-(méthylamino)oct-6-ène, de la S-éthylglycine, de la N-méthylglycine, de la N-méthyl-L-leucine, de la D-alanine, de la N-méthyl-L-valine, de NMeGly, de la L-ornithine, de la 3-anthranyloyl-L-alanine, de l'acide β-hydroxyglutamique, de l'aminoglycine, de (2S,3S)-Fmoc-AHPBA et de Fmoc-GAAP, où les acides aminés sont

éventuellement protégés sur les groupes terminaux N ou C et éventuellement sur le(s) groupe(s) réactif(s) dans la/les chaîne(s) latérale(s).

**9.** Procédé selon l'une quelconque des revendications 1 à 8, **caractérisé en ce que** les opérations des étapes c) et d) sur l'acide aminé terminal protégé du produit obtenu à chaque étape d) sont répétées au moins une fois de suite.

**10.** Procédé selon la revendication 9, **caractérisé en ce que** les opérations des étapes c) et d) sur l'acide aminé terminal protégé du produit obtenu à chaque étape d) sont répétées 1 à 100 fois de suite.

**11.** Procédé selon l'une quelconque des revendications 1 à 10, **caractérisé en ce que** l'étape e) est effectuée avec des esters d'hydroxysuccinimide ou, dans le cas d'un groupe fonctionnel acide carboxylique libre, avec des agents de couplage connus, ou avec des dérivés d'acide carboxylique, de préférence avec des chlorures d'acide carboxylique, des anhydrides d'acide carboxylique entraînant la formation de dérivés acylés.

**12.** Procédé selon la revendication 11, **caractérisé en ce que** l'étape e) est effectuée avec de la carboxyfluorescéine ou de l'anhydride acétique en présence d'un agent de couplage conduisant à la formation d'un dérivé marqué à la fluorescéine ou acétylé.

**13.** Procédé selon l'une quelconque des revendications 1 à 12, **caractérisé en ce que,** pour la synthèse de dérivés d'acide peptidique, le clivage à l'étape f) est effectué avec du fluorure d'hydrogène ou avec de l'acide trifluorométhanesulfonique dans le cas d'un lieur chlorométhylphényle, phényl-acétamidométhyle ; ou avec de l'acide trifluoroacétique en cas de lieur chlorure d'hydroxyméthylphényle, méthoxy-hydroxyméthylphényle, chlorotriphénylméthyle ; ou pour la synthèse de dérivés d'amide de peptide avec du fluorure d'hydrogène ou avec de l'acide trifluorométhanesulfonique dans le cas d'un lieur méthylbenzhydrylamine ; ou avec de l'acide trifluoroacétique dans le cas du lieur 2,4-diméthoxybenzhydrylamine ; ou avec une solution méthanolique d'ammoniac ou avec des amines primaires et secondaires en cas de lieur hydroxyméthylbenzamide ; ou pour la synthèse de dérivés peptidiques d'hydrazide avec des hydrazines, ou pour la synthèse de dérivés d'esters peptidiques avec des alcools, ou pour la synthèse de dérivés d'alcools peptidiques avec du borohydrure de lithium dans le cas du lieur hydroxyméthylbenzamide.

**14.** Procédé selon la revendication 13, **caractérisé en ce que,** pour la synthèse de dérivés d'acide peptidique dans le cas d'un lieur hydroxyméthylphényle et pour la synthèse de dérivés d'amide peptidique dans le cas d'un lieur 2,4-diméthoxybenzhydrylamine, le clivage à l'étape f) est effectué avec de l'acide trifluoroacétique.

**15.** Procédé selon l'une quelconque des revendications 1 à 14 pour la synthèse de peptides ALFE-$NH_2$, RLFE-$NH_2$, DLFE-$NH_2$, NLFE-$NH_2$, YLFE-$NH_2$, TLFE-$NH_2$, FLFE-$NH_2$, PLFE-$NH_2$, QLFE-$NH_2$, MLFE-$NH_2$, ELFE-$NH_2$, KLFE-$NH_2$, VLFE-NE2, GLFE-$NH_2$, CLFE-NH2, ILFE-$NH_2$, LLFE-$NH_2$, SLFE-$NH_2$, WLFE-$NH_2$, HLFE-$NH_2$, VQAAIDYING-$NH_2$, GILTVSVAV-$NH_2$, H-([1S,2R]-ACPC-[1R,2S]-ACPC)$_3$-$NH_2$, H-([1R,2S]-ACPC)$_6$-$NH_2$, [1S,2S]-ACHC-$\beta^3$-*h*Arg-([1S,2S]-ACHC)$_2$-$\beta^3$-*h*Asp-[1S,2S]-ACHC-Gly-Gly-Cys-$NH_2$, H-(GAAP)$_6$-$NH_2$, fluorescéinyl-YADAIFTNSYRKVLGQLSARKLLQDIMSR-$NH_2$, Ac-([1S,2S]-ACPC)$_{12}$-$NH_2$, H-AHPA-Leu-$NH_2$, **caractérisé en ce que** les matériaux de départ appropriés sont utilisés.

**16.** Procédé selon l'une quelconque des revendications 1 à 14 pour la synthèse d'un peptide choisi dans le groupe constitué de

$Arg_{5\text{-}8}$,
RQIKIWFQNRRMKWKK,
ACPC-ACPC,
ACHC-ACHC,
ACTH(1-24),
SYSMEHFRWGKPVGKKRRPVKVYP,

H-His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr-OH,

H-His-Ser-Asp-Gly-Thr-Phe-Thr-Ser-Glu-Leu-Ser-Arg-Leu-Arg-Asp-Ser-Ala-Arg-Leu-Gln-Arg-Leu-Leu-Gln-Gly-Leu-Val-$NH_2$,

piroGlu-His-Trp-Ser-Tyr-Gly-Leu-Arg-Pro-Gly-$NH_2$,
acétyl-D-3-(2'-naphthyl)-alanine-D-4-chlorophcnylalanine-D-3-(3'-pyridyl)-alanine-L-sérine-L-tyrozine-D-citrulline-L-leucine-L-arginine-L-proline-D-alamne-amide,
Pyr-His-Trp-Ser-Tyr-D-Lsu-Leu-Arg-Pro-NHEt,
(4R)-N-{4-[(2S)-2-{[(1R)-2-[4-(carbamoylamino)phenyl]-1-{[(1S)-1-{[(2S)-1-(2-{[(1R)-1-carbamoylethyl]carbamoyl}pyrrolidin-1-yl)-1-oxo-6-[(propane-2-yl)amino]hexane-2-yl] carbamoyl}-3-methylbutyl]carbamoyl}ethyl]carbamoyl}-2-[(2S)-2-[(2R)-2-[(2R)-3-(4-chlorophenyl)-2-[(2R)-2-acetamido-3-(naphthalene-2-yl)propaneamido]propaneamido]-3-(pyridin-3-yl)propane-amido]-3-hyroxypropaneamido]ethyl]phenyl}-2,6-dioxo-1,3-diazmane-4-carboxamide,
(2S)-N-[(2S)-1-[[(2S)-1-[[(2S)-1-[[(2S)-1-[[(2R)-1-[[(2S)-1-[[(2S)-5-(diaminomethylidene-amino)-1-[(2S)-2-(ethylcarbamoyl)pyrrolidin-1-yl]-1-oxopentane-2-yl]amino]-4-methyl-1-oxopentan-2-yl]amino]-3-(7*H*-indol-3-yl)-1-oxopropane-2-yl]amino]-3-(4-hyroxypheny1)-1-oxopropane-2-yl]amino]-3-hyroxy-1-oxopropane-2-yl]amino]-3-(1*H*-indol-3-yl)-1-oxopropane-2-yl]amino]-3-(*1H*-imidazol-5-yl)-1-oxo-propane-2-yl]-5-oxopirrolidin-2-carboxamide,
Pyr-His-Trp-Ser-Tyr-D-Trp-Leu-Arg-Pro-Gly-$NH_2$,
Pyr-His-Trp-Ser-Tyr-D-Ser(OtBu)-Leu-Arg-Pro-NH-NH-CO-$NH_2$,
Pyr-His-Trp-Ser-Tyr-D-Ser(OtBu)-Leu-Arg-Pro-NHEt,

H-Ser-Val-Ser-Glu-yle-Gln-Leu-Met-His-Agn-Leu-Gly-Lys-His-Leu-Asn-Ser-Met-Glu-Arg-Val-Glu-Trp-Leu-Arg-Lys-Lys-Leu-Gln-Asp-Val-His-Asn-Phe-OH,

SQEPPISLDLTFHLLREVLEMTKADQLAQQAHSNRKLLDIA,

HO-Tyr-Ala-Asp-Ala-yle-Phe-Thr-Asn-Scr-Tyr-Arg-Lys-Val-Leu-Gly-Gln-Leu-Ser-Ala-Arg-Lys-Leu-Leu-Gln-Asp-yle-Mel-Ser-Arg-Gln-Gh-Gly-Glu-Ser-Asn-Gln-Glu-Arg-Gly-Ala-Arg-Ala-Arg-Leu-$NH_2$,

HO-Gln-Leu-Ser-Ala-Arg-Lys-Leu-Leu-Gln-Asp-yle-Met-Ser-Arg-Gln-Gln-Gly-Glu-Ser-Asn-Gln-

Glu-Arg-Gly-Ala-Arg-Ala-Arg-Leu-$NH_2$,

Pyr-His-Pro-$NH_2$,
SDAAVDTSSEITTKDLKEKKEVVEEAEN,
RLDVY,
HGEGTFTSDLSKQMEEEAVRLFIEWLKNGGPSSGAPPPS,

H-His-Ala-Glu-Gly-Thr-Phe-Thr-Ser-Asp-Val-Ser-Ser-Tyr-Leu-Glu-Gly-Gln-Ala-Ala-Lys(γ-Glu-palmitoyl)-Glu-Phe-yle-Ala-Trp-Leu-Val-Arg-Gly-Arg-Gly-OH,

FPRPGGGGNGDFEEIPEEIL,
Pyr-His-Trp-Ser-Tyr-Gly-Leu-Arg-Pro-Gly-$NH_2$,
D-Arg-Arg-Pro-Hyp-Gly-Thi-Ser-D-Tic-Oic-Arg,
YADAIFTNSYRKVLGQLSARKLLQDIMSRQ,
acide [(1R)-3-méthyl-1-({(2S)-3-phényl-2-[(pyrazin-2-ylcarbonyl)amino]propanoyl}amino)butyl]-boronique,
N-[2,2-diméthyl-1-(méthylcarbamoyl)-propyl]-2-[hydroxy-(hydroxycarbamoyl)méthyl]-4-méthyl-pentaneamide,
GIGKFLKKAKKFGKAFVKILKK,
GIGKFLHSAKKFGKAFVGEIMNS,
IB367,

RGGLCYCRGRFCVCVGR,
LRWPWWPWRRK-$NH_2$,
(2*S*,3*R*)-AHPA-L-Leu,
(2*S*,3*R*)-AHPA-L-Pro-L-Pro-L-Ala-$NH_2$,
(2*S*,3*R*)-3-amino-2-hyroxy-5-méthylhexanoyl-L-Val-L-Val-L-Asp-OH,
(2*S*,3*R*)-AHPA-Val-Phe-OH,
(2*S*,3*R*)-Leu-Pro-Pro-OH,
3-amino-2-hyroxy-décanoyl-Ala-Val-NMeTyr-Tyr-OH,
CYIQNCPLG-$NH_2$,
3-Mercaptopropionyl-D-Tyr(Et)-yle-Thr-Asn-Cys-Pro-Orn-Gly-$NH_2$,
H-Cys-Tyr-Phe-Gln-Asn-Cys-Pro-Arg-Gly-$NH_2$,
*désamino*-Cys-Tyr-Phe-Gln-Asn-Cys-Pro-D-Arg-Gly-$NH_2$,
$N^6$-(aminoiminométhyl)-$N^2$-(3-mercapto-1-oxopropyl)-L-[ysyl-glycyl-L-$\alpha$-aspartyl-L-tryptophyl-L-prolyl-L-cistéinamide,
KCNTATCATQRLANFLVHSSNNPGPILPPTNVGSNTY-$NH_2$,
H-[(2*S*,3*R*,4*R*,6*E*)-3-hyroxy-4-méthyl-2-(méthylamino)oct-6-énoyl]-L-2-aminobutanoyl-N-méthylglycyl-*N*-méthyl-L-leucyl-L-valyl-*N*-méthyl-L-leucy-L-alanyl-D-alanyl-*N*-méthyl-L-leucyl-*N*-méthyl-L-leucyl-*N*-méthyl-L-valine-OH,
Ac-YTSLIHSLIEESQNQQEKNEQELLELDKWASLWNWF-$NH_2$,
H-Val-Pro-NMeGly-OH,
H-D-Phe-Cys-Phe-D-Trp-Lys-Thr-Cys-Thr(ol),
N-décanoyl-L-triptophyl-L-asparaginyl-L-aspartyl-L-tréonylglicyl-L-omityl-L-aspartyl-D-alanyl-L-aspartylglicyl-D-séryl-thréo-3-méthyl-L-glutamyl-3-anthranyloyl-L-alanine,
H-Val-Orn-Leu-D-Phe-Pro-Val-Orn-Leu-D-Phe-Pro-OH, **caractérisé en ce que** les matériaux de départ appropriés sont utilisés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3531258 A, Merrifield
- US 4746490 A
- US 5288464 A
- US 5380495 A
- US 6028172 A
- US 7550560 B
- US 4192798 A
- WO 8203077 A


### Non-patent literature cited in the description


- **BERND GRONER.** Peptides as Drugs: Discovery and Development. Wiley-VCH, 2009
- **C.M.B. EDWARDS ; M.A. COHEN ; S.R. BLOOM.** *Q. J. Med.,* 1999, vol. 92, 1-4
- **CYNTHIA L. STEVENSON.** Current Pharmaceutical. *Biotechnology,* 2009, vol. 10, 122-137
- **SHAK, S.** *SEMINARS IN ONCOLOGY,* 1999, vol. 26, 71-77
- **FISCHER, G ; SCHIRRMACHER, R ; WANGLER, B ; WANGLER, C.** *CHEMMEDCHEM,* 2013, vol. 8, 883-890
- **BACSA, BERNADETT ; BOSZE, SZILVIA ; KAPPE, C. OLIVER.** *JOURNAL OF ORGANIC CHEMISTRY,* 2010, vol. 75, 2103-2106
- **V.R. PATTABIRAMAN ; J. W. BODE.** *Nature,* 2011, vol. 480, 471-479
- **MURRAY GOODMAN ; CLAUDIO TONIOLO ; LUIS MORODER.** *Synthesis of Peptides and Peptidomimetics (Methods in Organic Chemistry) Houben-Weyl,* 2002
- **ANDREW B. HUGHES.** Amino Acids, Peptides and Proteins in Organic Chemistry. Wiley-VCH, 2011, vol. 1-5
- **R. B. MERRIFIELD.** *J. Am. Chem. Soc.,* 1963, vol. 85, 2149-2154
- **NORBERT SEWALD ; HANS-DIETER JAKUBKE.** Peptides: Chemistry and Biology. Wiley-VCH, 2002
- **STACEY A. PALASEK ; ZACHARY J. COX ; JONATHAN M. COLLINS.** *J. Pept. Sci.,* 2007, vol. 13, 143-148
- Amino Acids. **ANDREW B. HUGHES.** Peptides and Proteins in Organic Chemistry. Wiley-VCH, 2011, vol. 1-5
- **BERNADETT BACSA ; C OLIVER KAPPE.** *Nature Protocols,* 2007, vol. 2, 2222-2227, http://www.dddmag.com/articles/2012/12/ microwave-assisted-peptide-synthesis
- **C. M. GOODMAN ; S. CHOI ; S. SHANDLER ; W. F. DEGRADO.** *Nat. Chem. Biol.,* 2007, vol. 3, 252-262
- **D. SEEBACH ; J. GARDINER.** *Acc. Chem. Res.,* 2008, vol. 41, 1366-1375
- **JUSTIN K. MURRAY ; BILAL FAROOQI ; JACK D. SADOWSKY ; MARK SCALF ; WESLEY A. FREUND ; LLOYD M. SMITH ; JIANDONG CHEN ; SAMUEL H. GELLMAN.** *J. AM. CHEM. SOC.,* 2005, vol. 127, 13271-13280
- **NORBERT SEWALD.** Hans-Dieter Jakubke Peptides: Chemistry and Biology. Wiley-VCH, 2002
- **FORRO, E. ; FULOP, F.** *Chem. Eur. J.,* 2006, vol. 12, 2587-2592
- **MURRAY GOODMAN ; CLAUDIO TONIOLO ; LUIS MORODER.** *Synthesis of Peptides and Peptidomimetics (Methods in Organic Chemistry) [Houben-Weyl,* 2002
- **ALESIA A. TIETZE ; PASCAL HEIMER ; ANNEGRET STARK ; DIANA IMHOF.** *Molecules,* 2012, vol. 17 (4158), 8-4185
- **MURRAY GOODMAN ; CLAUDIO TONIOLO ; LUIS MORODER.** *Synthesis of Peptides and Peptidomimetics (Methods in Organic Chemistry) Houben-Weyl,* 2002, vol. E 22 a-d
- Amino Acids. **ANDREW B. HUGHES.** Peptides and Proteins in Organic Chemistry. Wiley-VCH, 2011, vol. 5
- **NORBERT SEWALD.** Hans-Dieter Jakubke: Peptides: Chemistry and Biology. Wiley-VCH, 2002, 229
- **LES P. MIRANDA ; PAUL F. ALEWOOD.** *Proc. Natl. Acad. Sci. USA,* 1999, vol. 96, 1181-1186
- **BERNADETT BACSA ; KATA HORVÁTI ; SZILVIA BŐSZE ; FRITZ ANDREAE ; C. OLIVER KAPPE.** *Journal of Organic Chemistry,* 2008, vol. 73, 7532-7542

- **RICHARD P. CHENG ; SAMUEL H. GELLMAN ; WILLIAM F. DEGRADO.** *Chem. Rev.,* 2001, vol. 101, 3219-3232
- **FULOP, L. ; MANDITY, I. M. ; JUHASZ, G. ; SZEGEDI, V. ; HETENYI, A. ; WEBER, E. ; BOZSO, Z. ; SIMON, D. ; BENKO, R. ; KIRALY, Z.** *Plos One,* 2012, 7
- **BEATA KOLESINSKA ; DOMINIKA J. PODWYSOCKA ; MAGNUS A. RUEPING ; DIETER SEEBACH ; FAUSTIN KAMENA ; PETER WALDE ; MARKUS SAUER ; BARBARA WINDSCHIEGL ; MIRA MEYER-ACS ; MARC VOR DER BRÜGGEN.** *CHEMISTRY & BIODIVERSITY,* 2013, vol. 10, 1-38